# EUROPÄISCHE PATENTSCHRIFT

(11) **EP 1 567 613 B1**
(45) Veröffentlichungstag und Bekanntmachung des Hinweises auf die Patenterteilung: **07.03.2007**
(21) Anmeldenummer: 03779874.1
(22) Anmeldetag: 05.11.2003
(51) Int. Cl.: C09K 19/32, C09K 19/34

(54) **CYCLOBUTANDERIVATE**
CYCLOBUTANE DERIVATIVES
DERIVES DE CYCLOBUTANE

(30) Priorität: 02.12.2002 DE 10256172
(43) Veröffentlichungstag der Anmeldung: 31.08.2005
(73) Patentinhaber: Merck Patent GmbH, 64293 Darmstadt (DE)
(72) Erfinder: TAUGERBECK, Andreas, 64285 Darmstadt (DE); HECKMEIER, Michael, 69502 Hemsbach (DE); KIRSCH, Peer, 64342 Seeheim-Jugenheim (DE); LÜSSEM, Georg, 85238 Petershausen (DE); POETSCH, Eike, 64367 Mühltal (DE)
(86) Internationale Anmeldenummer: PCT/EP2003/012511
(87) Internationale Veröffentlichungsnummer: WO 2004/050796

(56) Entgegenhaltungen:
- WO-A-02/48073
- DE-A- 3 717 484
- DE-A- 4 303 634
- DE-A- 4 408 418
- DE-A- 19 755 245
- US-A- 4 853 151
- US-A- 5 445 764
- US-A- 6 146 719
- US-A1- 2002 030 180
- PATENT ABSTRACTS OF JAPAN vol. 1999, no. 08, 30. Juni 1999 (1999-06-30) -& JP 11 080049 A (CHISSO CORP), 23. März 1999 (1999-03-23)

## Beschreibung

Die vorliegende Erfindung betrifft Cyclobutanderivate, die sowohl eine CF₂O-Brücke als Bestandteil ihres mesogenen Grundgerüsts als auch eine Butan-1,4-diyl-Gruppe als Teil einer Spiroalkaneinheit enthalten, ihre Verwendung als Komponente(n) in flüssigkristallinen Medien sowie Flüssigkristall- und elektrooptische Anzeigeelemente, die diese erfindungsgemäßen flüssigkristallinen Medien enthalten.

Die erfindungsgemäßen Cyclobutanderivate können als Komponenten flüssigkristalliner Medien verwendet werden, insbesondere für Displays, die auf dem Prinzip der verdrillten Zelle, dem Guest-Host-Effekt, dem Effekt der Deformation aufgerichteter Phasen DAP oder ECB (Electrically controlled birefringence), dem IPS-Effekt (In Plane Switching) oder dem Effekt der dynamischen Streuung beruhen.

Die bisher für diesen Zweck eingesetzten Substanzen haben stets gewisse Nachteile, beispielsweise zu geringe Stabilität gegenüber der Einwirkung von Wärme, Licht oder elektrischen Feldern, ungünstige elastische und/oder dielektrische Eigenschaften.

In der US 2002/0030180 A1 wird ein flüssigkristallines Medium basierend auf einer Mischung von polaren Verbindungen mit positiver dielektrischer Anisotropie offenbart, welche eine oder mehrere Verbindungen der Formel 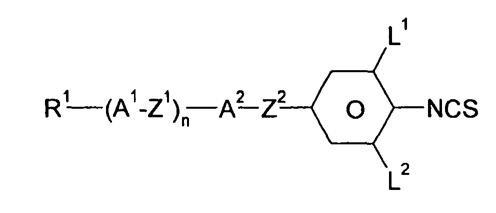 enthält, worin R¹, A¹, A², Z¹, Z², L¹, L² und n die in diesem Dokument angegebenen Bedeutungen aufweisen.

Der Erfindung lag somit die Aufgabe zugrunde, neue stabile flüssigkristalline oder mesogene Verbindungen aufzufinden, die als Komponenten flüssigkristalliner Medien, insbesondere für TN-, STN-, IPS- und TFT-Displays, geeignet sind.

Eine weitere Aufgabe der vorliegenden Erfindung war es, flüssigkristalline Verbindungen bereitzustellen, die eine niedrige Rotationsviskosität aufweisen, in Flüssigkristallmischungen zu einer Verbesserung der Tieftemperaturstabilität führen und sich einfach synthetisieren lassen. Insbesondere durch die Verringerung der Rotationsviskosität sollten sich deutlich geringere Schaltzeiten realisieren lassen.

Überraschenderweise wurde gefunden, dass die erfindungsgemäßen Cyclobutanderivate vorzüglich als Komponenten flüssigkristalliner Medien geeignet sind. Mit ihrer Hilfe lassen sich stabile, flüssigkristalline Medien, insbesondere geeignet für TFT- oder STN-Displays, erhalten.

Durch geeignete Wahl der Ringglieder und/oder der terminalen Substituenten lassen sich die physikalischen Eigenschaften der erfindungsgemäßen Cyclobutanderivate in weiten Bereichen variieren. So ist es beispielsweise möglich, erfindungsgemäße Cyclobutanderivate mit sehr kleinen Werten der optischen Anisotropie oder mit geringen positiven bis stark positiven Werten der dielektrischen Anisotropie zu erhalten.

Insbesondere zeichnen sich die erfindungsgemäßen Cyclobutanderivate durch hohe Klärpunkte bei gleichzeitig unerwartet niedriger Rotationsviskosität aus.

Flüssigkristalline.Medien mit sehr kleinen Werten der optischen Anisotropie sind insbesondere für reflektive und transflektive Anwendungen von Bedeutung, d.h. solche Anwendungen, bei denen das jeweilige LCD keine oder nur unterstützende Hintergrundbeleuchtung erfährt.

Mit der Bereitstellung der erfindungsgemäßen Cyclobutanderivate wird ganz allgemein die Palette der flüssigkristallinen Substanzen, die sich unter verschiedenen, anwendungstechnischen Gesichtspunkten zur Herstellung flüssigkristalliner Gemische eignen, erheblich verbreitert.

Die erfindungsgemäßen Cyclobutanderivate besitzen einen breiten Anwendungsbereich. In Abhängigkeit von der Auswahl der Substituenten können diese Verbindungen als Basismaterialien dienen, aus denen flüssigkristalline Medien zum überwiegenden Teil zusammengesetzt sind; es können aber auch den erfindungsgemäßen Cyclobutanderivaten flüssigkristalline Basismaterialien aus anderen Verbindungsklassen zugesetzt werden, um beispielsweise die dielektrische und/oder optische Anisotropie eines solchen Dielektrikums zu beeinflussen und/oder um dessen Schwellenspannung und/oder dessen Viskosität zu optimieren.

Die erfindungsgemäßen Cyclobutanderivate sind in reinem Zustand farblos und bilden flüssigkristalline Mesophasen in einem für die elektrooptische Anwendung günstig gelegenen Temperaturbereich. Chemisch, thermisch und gegen Licht sind sie stabil.

Gegenstand der vorliegenden Erfindung sind somit Cyclobutanderivate der Formel I 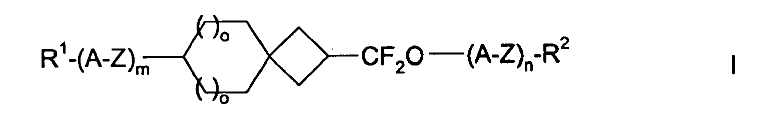 worin
- R¹, R²: jeweils unabhängig voneinander, gleich oder verschieden H, Halogen (F, Cl, Br, I) oder einen linearen oder verzweigten, gegebenenfalls chiralen, unsubstituierten, ein- oder mehrfach durch Halogen substituierten Alkyl- oder Alkoxyrest mit 1 bis 15 C-Atomen, worin auch eine oder mehrere CH₂-Gruppen jeweils unabhängig voneinander durch -O-, -S-, -CO-, -CO-O-, -O-CO-, -O-CO-O-, -CH=CH-, -CH=CF-, -CF=CF-, -C≡C- oder -◇- so ersetzt sein können, dass Heteroatome nicht direkt miteinander verknüpft sind, -CN, -SCN, -SF₅, -SCF₃, -CF₃, -CF=CF₂, -CF₂CF₂CF₃, -OCF₃, -OCHF₂, -CF₂CH₂CF₃ oder -OCH₂CF₂CHFCF₃,
- A: jeweils unabhängig voneinander, gleich oder verschieden
a) trans-1,4-Cyclohexylen, worin auch eine oder mehrere nicht benachbarte CH₂-Gruppen durch -O- und/oder -S- ersetzt sein können und worin auch ein oder mehrere H-Atome durch F ersetzt sein können,
b) 1,4-Phenylen, worin eine oder zwei CH-Gruppen durch N ersetzt sein können und worin auch ein oder mehrere H-Atome durch Halogen (F, Cl, Br, I), -CN, -CH₃, -CHF₂, -CH₂F, -OCH₃, -OCHF₂ oder -OCF₃ ersetzt sein können,
c) einen Rest aus der Gruppe Bicyclo[1.1.1]pentan-1,3-diyl, Bicyclo[2.2.2]-octan-1,4-diyl, Spiro[3.3]heptan-2,6-diyl, Naphthalin-2,6-diyl, Decahydronaphthalin-2,6-diyl, 1,2,3,4-Tetrahydronaphthalin-2,6-diyl und Piperidin-1,4-diyl, oder
d) 1,4-Cyclohexenylen,
- Z: jeweils unabhängig voneinander, gleich oder verschieden -O-, -CH₂O-, -OCH₂-, -CO-O-, -O-CO-, -CF₂O-, -OCF₂-, -CF₂CF₂-, -CH₂CF₂-, -CF₂CH₂-, -CH₂CH₂-, -CH=CH-, -CH=CF-, -CF=CH-, -CF=CF-, -CF=CF-COO-, -O-CO-CF=CF-, -C≡C- oder eine Einfachbindung,
- m, n: unabhängig voneinander, gleich oder verschieden 0, 1 oder 2, vorzugsweise m = 0 oder 1 und n = 1 oder 2, und
- o: 0 oder 1,
bedeuten.

Ein weiterer Gegenstand der vorliegenden Erfindung ist die Verwendung von Verbindungen der Formel I als Komponente(n) in flüssigkristallinen Medien.

Ebenfalls Gegenstand der vorliegenden Erfindung sind flüssigkristalline Medien mit mindestens zwei flüssigkristallinen Komponenten, welche mindestens eine Verbindung der Formel I enthalten.

Gegenstand der vorliegenden Erfindung sind auch Flüssigkristall-Anzeigeelemente, insbesondere elektrooptische Anzeigeelemente, welche als Dielektrikum ein erfindungsgemäßes, flüssigkristallines Medium enthalten.

Besonders bevorzugt sind reflektive und transflektive Flüssigkristall-Anzeigeelemente sowie andere Flüssigkristall-Anzeigen mit niedriger Doppelbrechung Δn, sogenannte "low Δn mode displays", wie z.B. reflektive und transflektive TN-Anzeigen.

Die Bedeutung der Formel I schließt alle Isotope der in den Verbindungen der Formel I gebundenen chemischen Elemente ein. In enantiomerenreiner oder -angereicherter Form eignen sich die Verbindungen der Formel I auch als chirale Dotierstoffe und generell zur Erzielung chiraler Mesophasen.

Vor- und nachstehend haben R¹, R², A, Z, m, n und o die angegebene Bedeutung, sofern nicht ausdrücklich etwas anderes vermerkt ist. Kommen die Reste A und Z mehrfach vor, so können sie unabhängig voneinander gleiche oder verschiedene Bedeutungen annehmen.

Bevorzugt sind Verbindungen der Formel I, worin R¹ H oder einen linearen Alkylrest mit 1 bis 10 C-Atomen bedeutet.

Ebenfalls bevorzugt sind Verbindungen der Formel I, worin R² H, einen linearen Alkoxyrest mit 1 bis 10 C-Atomen, -F, -Cl, -CF₃, -OCF₃, -OCHF₂, -CN oder -SF₅, besonders bevorzugt -OC₂H₅, -F, -CF₃, -OCF₃ oder -CN, bedeutet.

Bevorzugte Verbindungen der Formel I sind Verbindungen der Teilformel Ia 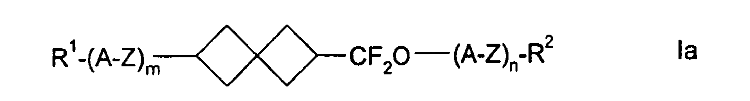 und Verbindungen der Teilformel Ib 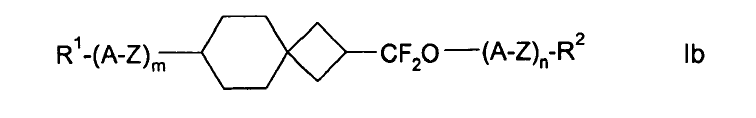 worin R¹, R², A, Z, m und n die oben angegebenen Bedeutungen besitzen.

Z bedeutet vorzugsweise -CH₂CH₂-, -CH=CH-, -C≡C-, -CF₂CF₂-, -CF=CF-, -CF₂O- oder eine Einfachbindung, besonders bevorzugt eine Einfachbindung.

Der Einfachheit halber bedeuten im folgenden Cyc einen 1,4-Cyclohexylenrest, Che einen 1,4-Cyclohexenylenrest, Dio einen 1,3-Dioxan-2,5-diylrest, Dit einen 1,3-Dithian-2,5-diylrest, Phe einen 1,4-Phenylenrest, Pyd einen Pyridin-2,5-diylrest, Pyr einen Pyrimidin-2,5-diylrest und Bco einen Bicyclo-(2,2,2)-octylenrest, Dec einen Decahydronaphthalin-2,6-diylrest, wobei Cyc und/oder Phe unsubstituiert oder ein- oder mehrfach durch CH₃, Cl, F oder CN substituiert sein können.

A bedeutet vorzugsweise Phe, Cyc, Che, Pyd, Pyr oder Dio, und besonders bevorzugt Phe oder Cyc.

Phe ist vorzugsweise

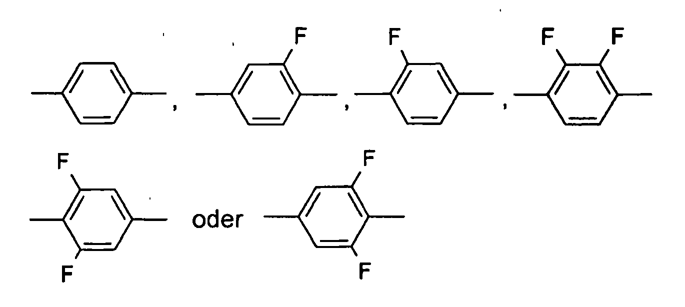

Die Begriffe 1,3-Dioxan-2,5-diyl und Dio umfassen jeweils die beiden Stellungsisomeren

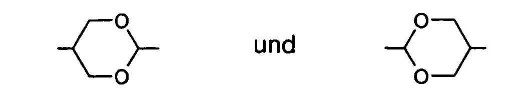

Die Cyclohexen-1,4-diyl-Gruppe hat vorzugsweise folgende Strukturen:

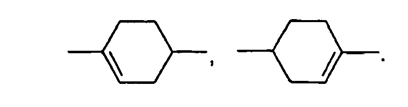

Besonders bevorzugte Verbindungen der Formel Ia umfassen die folgenden Formeln:

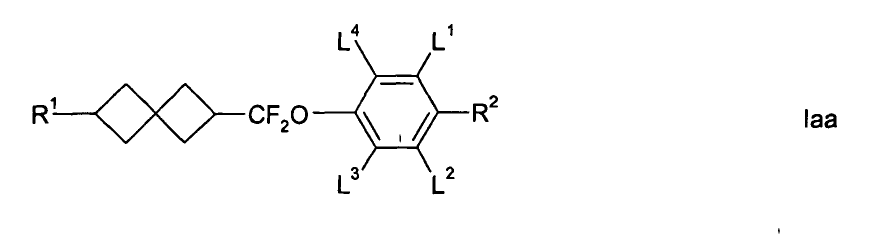

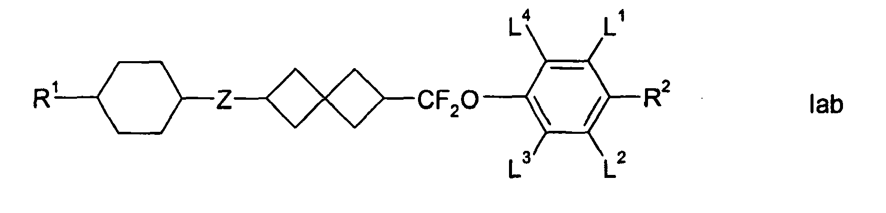

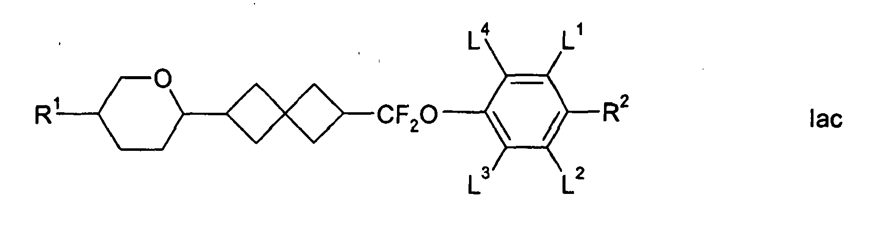

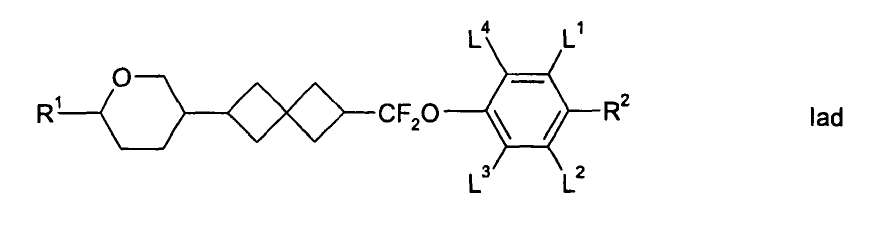

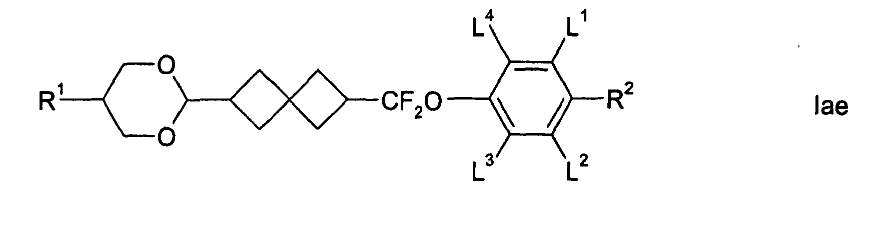

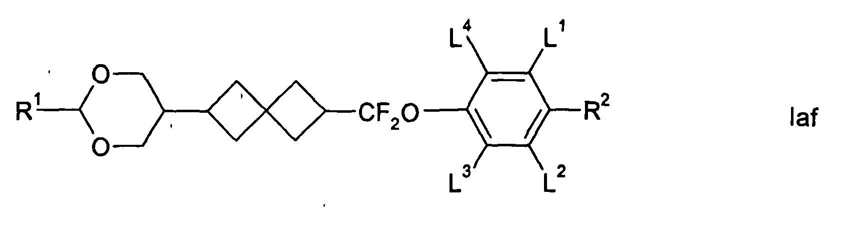

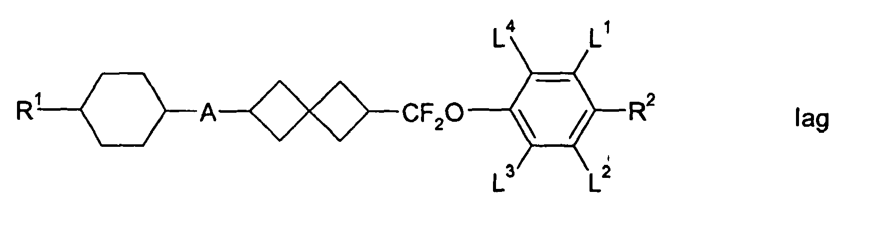

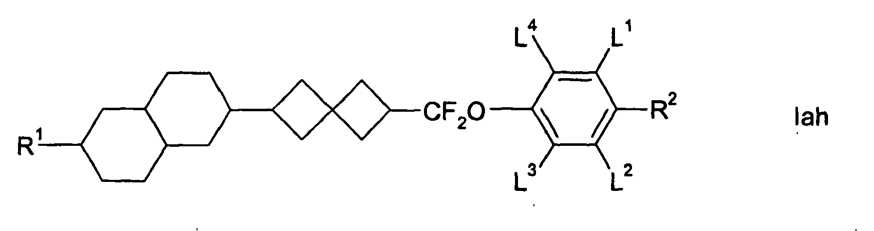

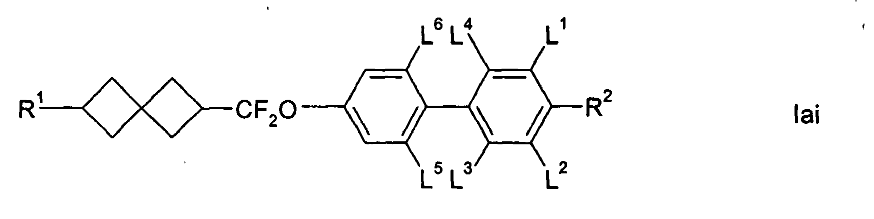

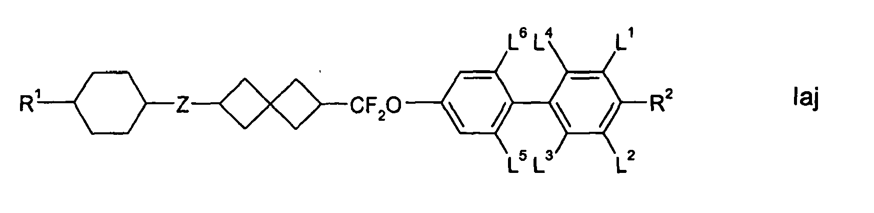

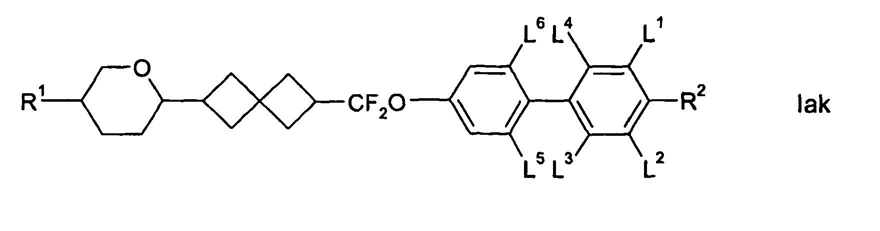

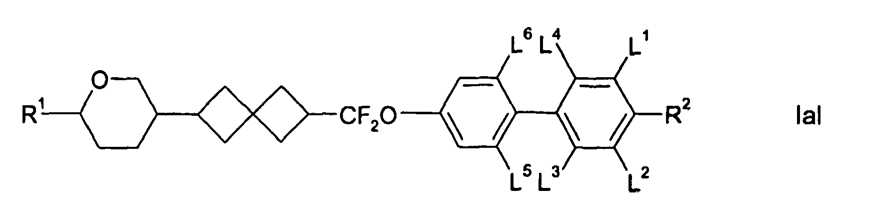

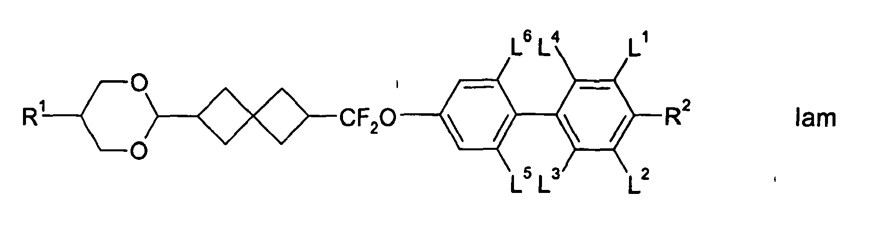

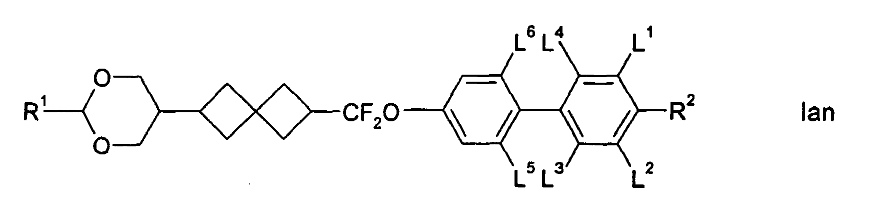

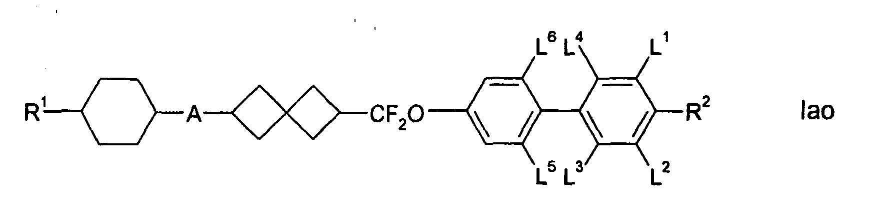

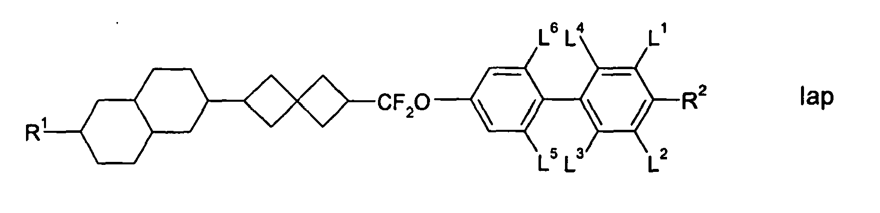

Besonders bevorzugte Verbindungen der Formel Ib umfassen die folgenden Formeln: 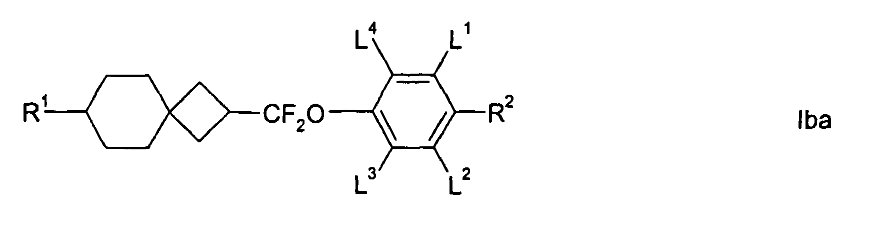 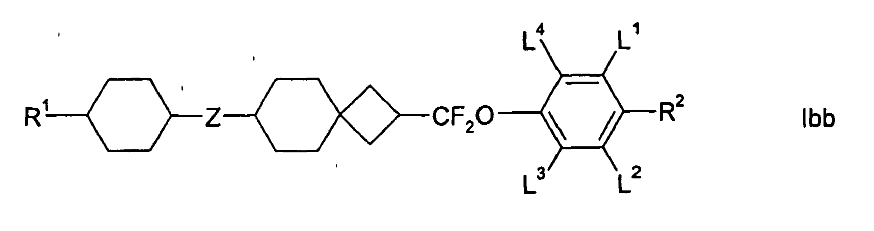 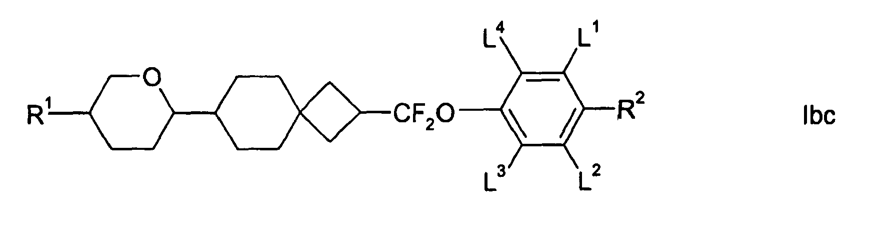 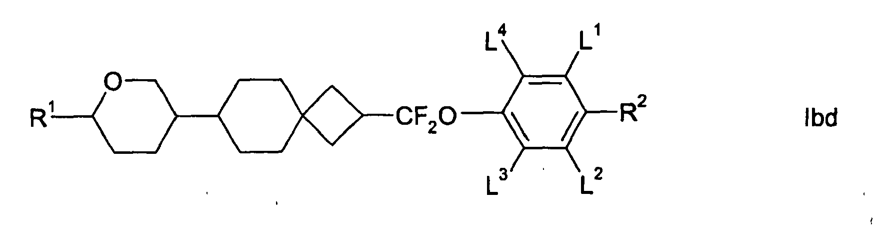 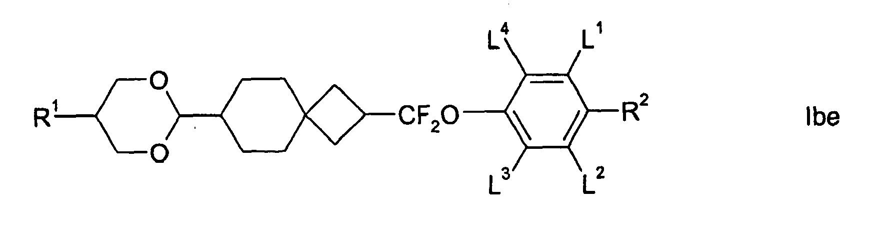 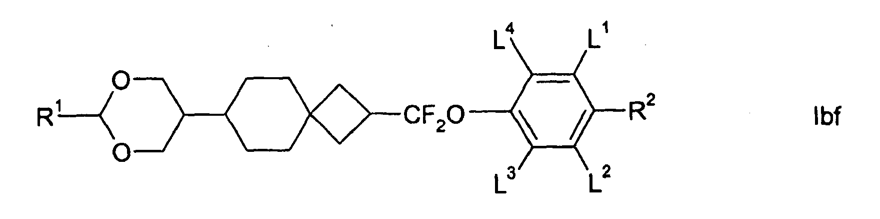 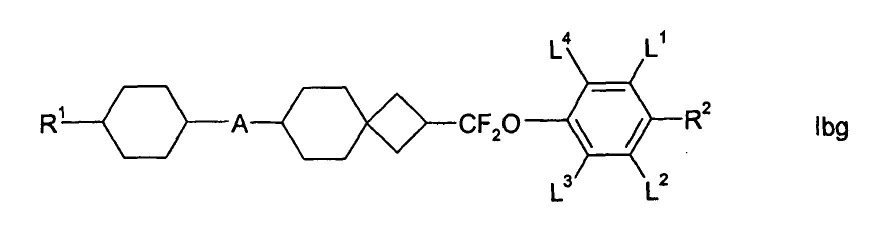 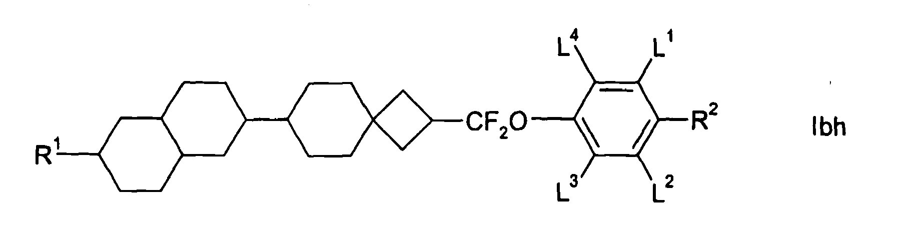 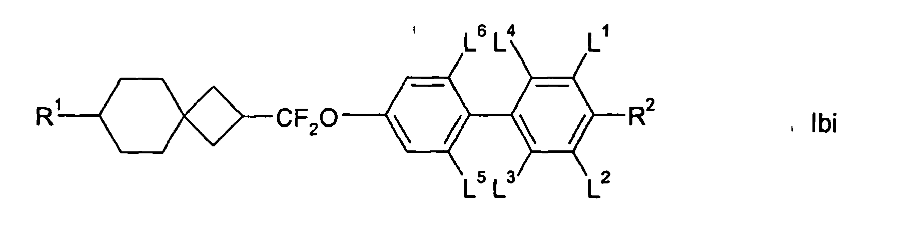 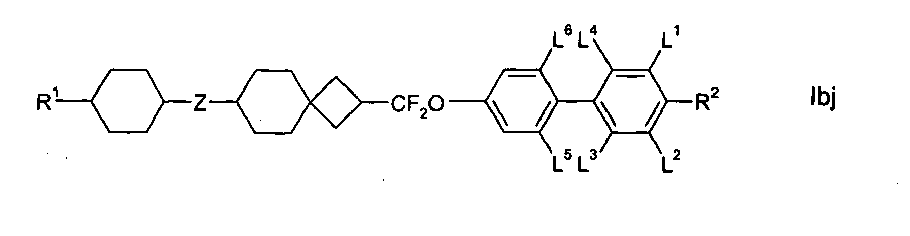 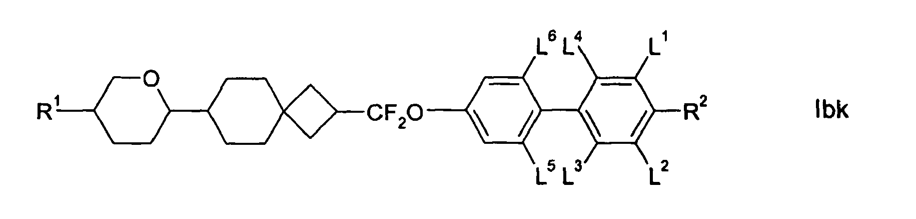 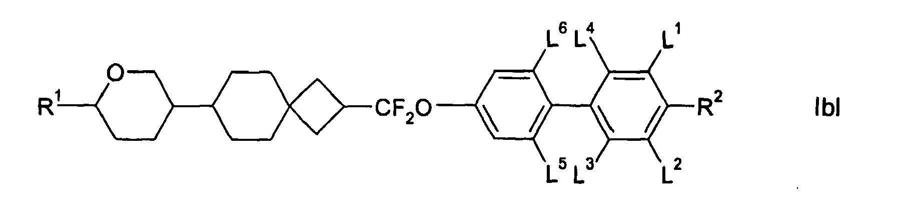 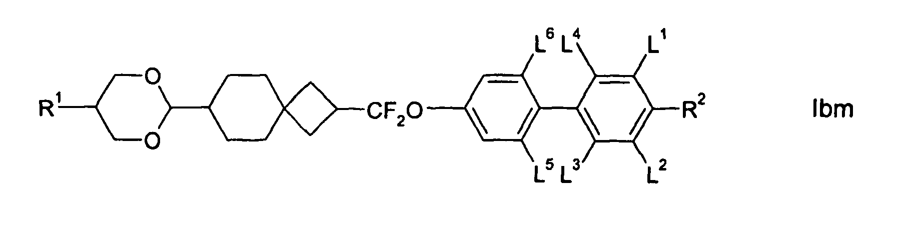 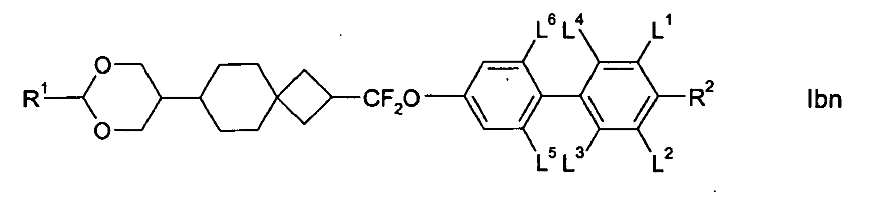 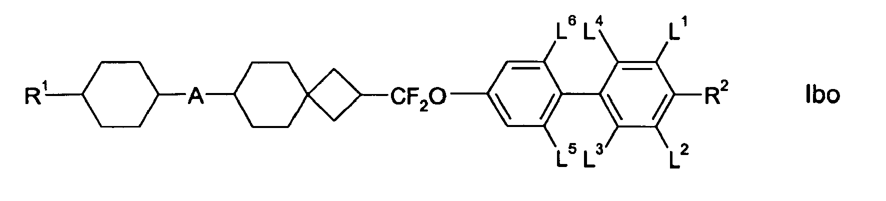 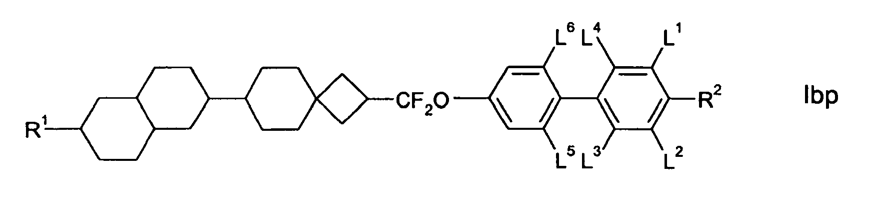 worin R¹, R², A und Z die oben angegebenen Bedeutungen besitzen und L¹, L², L³, L⁴, L⁵ und L⁶ unabhängig voneinander, gleich oder verschieden H oder F bedeuten.

Bevorzugt sind Verbindungen der Formel laa bis lap sowie Iba bis Ibp, worin R¹ H oder einen linearen Alkyl- bzw. Alkoxyrest mit 1 bis 10 C-Atomen oder Alkenyl bzw. Alkenyloxy mit 2 bis 10 C-Atomen bedeutet.

Ebenfalls bevorzugt sind Verbindungen der Formel Iaa bis lap sowie Iba bis Ibp, worin R² -F, -CF₃, -OCF₃, -CN, -SF₅ oder -OC₂H₅ bedeutet.

Besonders bevorzugt sind Verbindungen der Formel Iaa bis lah sowie Iba bis Ibh, worin R² -F, -CF₃, -OCF₃, -CN oder -SF₅ bedeutet, L¹ und L² unabhängig voneinander, gleich oder verschieden F oder H bedeuten und L³ und L⁴ H bedeuten.

Besonders bevorzugt sind Verbindungen der Formel laa bis lah sowie Iba bis Ibh, worin R² -OC₂H₅ bedeutet, L² und L³ unabhängig voneinander, gleich oder verschieden F oder H bedeuten und L¹ und L⁴ H bedeuten.

Besonders bevorzugt sind Verbindungen der Formel lai bis lap sowie Ibi bis Ibp, worin R² -F, -CF₃, -OCF₃, -CN oder-SF₅ bedeutet, L¹, L², L⁵ und L⁶ unabhängig voneinander, gleich oder verschieden F oder H bedeuten und L³ und L⁴ H bedeuten.

Besonders bevorzugt sind Verbindungen der Formel lai bis lap sowie Ibi bis Ibp, worin R² -OC₂H₅ bedeutet, L², L³ und L⁶ unabhängig voneinander, gleich oder verschieden F oder H bedeuten und L¹, L⁴ und L⁵ H bedeuten.

In den Verbindungen der Formeln lab, Iaj, Ibb und Ibj bedeutet Z vorzugsweise -CH₂CH₂-, -CF₂CF₂-, -CF₂O- oder eine Einfachbindung, besonders bevorzugt eine Einfachbindung.

In den Verbindungen der Formeln lag, lao, Ibg und Ibo bedeutet A vorzugsweise

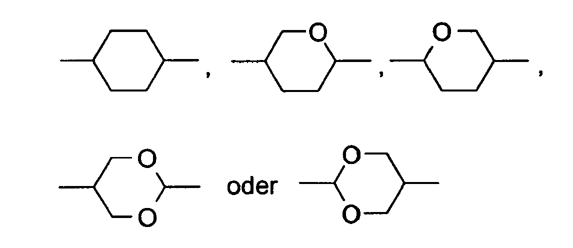

Falls R¹ und/oder R² in den vor- und nachstehenden Formeln einen Alkylrest bedeutet, so kann dieser geradkettig oder verzweigt sein. Besonders bevorzugt ist er geradkettig, hat 2, 3, 4, 5, 6 oder 7 C-Atome und bedeutet demnach Ethyl, Propyl, Butyl, Pentyl, Hexyl oder Heptyl, ferner Methyl, Octyl, Nonyl, Decyl, Undecyl, Dodecyl, Tridecyl, Tetradecyl oder Pentadecyl.

Falls R¹ und/oder R² einen Alkylrest bedeutet, in dem eine CH₂-Gruppe durch -O- ersetzt ist, so kann dieser geradkettig oder verzweigt sein. Vorzugsweise ist er geradkettig und hat 1 bis 10 C-Atome. Besonders bevorzugt ist die erste CH₂-Gruppe dieses Alkylrestes durch -O- ersetzt, so dass der Rest R¹ und/oder R² die Bedeutung Alkoxy erhält und insbesondere Methoxy, Ethoxy, Propoxy, Butoxy, Pentyloxy, Hexyloxy, Heptyloxy, Octyloxy oder Nonyloxy bedeutet.

Weiterhin kann auch eine CH₂-Gruppe an anderer Stelle durch -O- ersetzt sein, so dass der Rest R¹ und/oder R² vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl bedeutet.

Falls R¹ und/oder R² einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach Vinyl, Prop-1- oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Bevorzugte Alkenylgruppen sind C₂-C₇-1 E-Alkenyl, C₄-C₇-3E-Alkenyl, C₅-C₇-4-Alkenyl, C₆-C₇-5-Alkenyl, und C₇-6-Alkenyl, besonders bevorzugt C₂-C₇-1E-Alkenyl, C₄-C₇-3E-Alkenyl und C₅-C₇-4-Alkenyl.

Beispiele besonders bevorzugter Alkenylgruppen sind Vinyl, 1E-Propenyl, 1E-Butenyl, 1E-Pentenyl, 1E-Hexenyl, 1E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl und 6-Heptenyl. Gruppen mit bis zu 5 Kohlenstoffatomen sind insbesondere bevorzugt.

Falls R¹ und/oder R² einen Alkylrest bedeutet, in dem eine CH₂-Gruppe durch -O- und eine durch -CO- ersetzt ist, so sind diese bevorzugt benachbart. Somit beeinhalten diese eine Acyloxygruppe -CO-O- oder eine Oxycarbonylgruppe -O-CO-. Besonders bevorzugt sind diese geradkettig und haben 2 bis 6 C-Atome.

Sie bedeuten demnach insbesondere Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl und 4-(Methoxycarbonyl)butyl.

Falls R¹ und/oder R² einen Alkylrest bedeutet, in dem eine CH₂-Gruppe durch unsubstituiertes oder substituiertes -CH=CH- und eine benachbarte CH₂-Gruppe durch CO, CO-O oder O-CO- ersetzt ist, so kann dieser geradkettig oder verzweigt sein. Vorzugsweise ist er geradkettig und hat 4 bis 13 C-Atome. Er bedeutet demnach besonders bevorzugt Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl und 9-Methacryloyloxynonyl.

Falls R¹ und/oder R² einen einfach durch CN oder CF₃ substituierten Alkyl- oder Alkenylrest bedeutet, so ist dieser Rest vorzugsweise geradkettig und die Substitution durch CN oder CF₃ in ω-Position.

Falls R¹ und/oder R² einen mindestens einfach durch Halogen substituierten Alkylrest bedeutet, so ist dieser Rest vorzugsweise geradkettig. Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verbindungen der Formel I mit verzweigter Flügelgruppe R¹ und/oder R² können gelegentlich, wegen einer besseren Löslichkeit in den üblichen, flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien.

Verzweigte Gruppen dieser Art enthalten in der Regel nicht mehr als eine Kettenverzweigung. Bevorzugte verzweigte Reste R¹ und/oder R² sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentyloxy, 3-Methylpentyloxy, 2-Ethylhexyloxy, 1-Methylhexyloxy und 1-Methylheptyloxy.

Formel I umfasst sowohl die Racemate dieser Verbindungen als auch die optischen Antipoden sowie deren Gemische.

Unter den Verbindungen der Formel I sowie den Unterformeln sind diejenigen bevorzugt, in denen mindestens einer der darin enthaltenden Reste eine der angegebenen bevorzugten Bedeutungen hat.

Die Verbindungen der Formel I werden nach an sich bekannten Methoden dargestellt, wie sie in der Literatur (z.B. in den Standardwerken wie Houben-Weyl, Methoden der Organischen Chemie, Georg-Thieme-Verlag, Stuttgart) beschrieben sind und zwar unter Reaktionsbedingungen, die für die genannten Umsetzungen bekannt und geeignet sind. Dabei kann man von an sich bekannten, hier nicht näher erwähnten Varianten Gebrauch machen.

Die Verbindungen der Formel I können z.B. nach den folgenden Reaktionsschemata oder in Analogie dazu hergestellt werden. Weitere Synthesemethoden finden sich in den Beispielen.

In den Schemata 1 bis 4 haben R¹, R², A, Z, n, m und o die vorstehend angegebene Bedeutung. L¹, L², L³, L⁴, L⁵ und L⁶ bedeuten unabhängig voneinander, gleich oder verschieden H oder F.

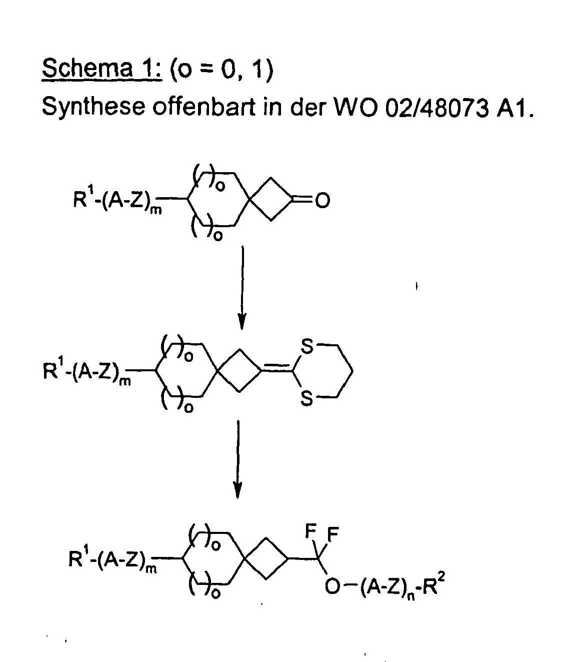

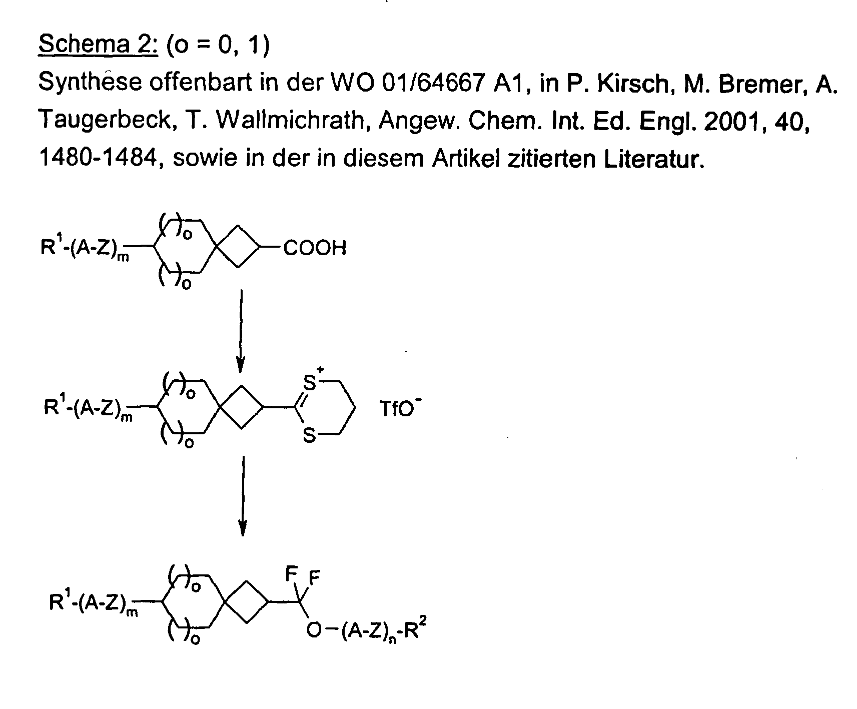

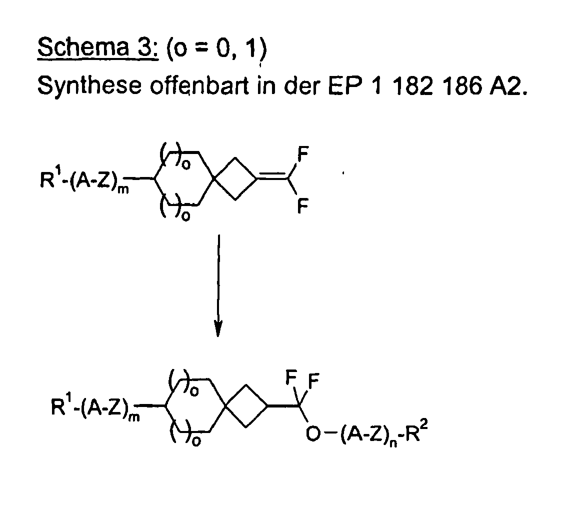

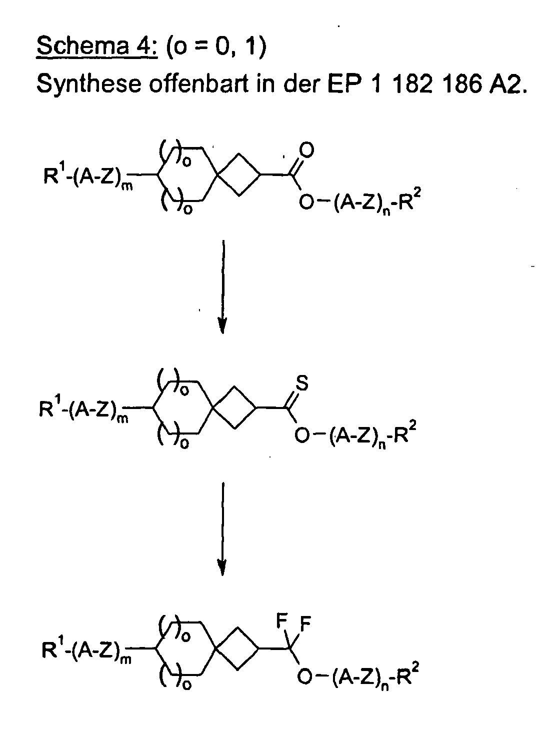

Die Ausgangsmaterialien sind entweder bekannt oder können in Analogie zu bekannten Verbindungen hergestellt werden.

Gegebenenfalls können die Ausgangsstoffe auch in situ gebildet werden, derart, dass man sie aus dem Reaktionsgemisch nicht isoliert, sondern sofort weiter zu den Verbindungen der Formel I umsetzt.

Die erfindungsgemäßen, flüssigkristallinen Medien enthalten vorzugsweise neben einer oder mehreren erfindungsgemäßen Verbindungen als weitere Bestandteile 2 bis 40, besonders bevorzugt 4 bis 30 Komponenten. Insbesondere enthalten diese Medien neben einer oder mehreren erfindungsgemäßen Verbindungen 7 bis 25 Komponenten. Diese weiteren Bestandteile werden vorzugsweise ausgewählt aus nematischen oder nematogenen (monotropen oder isotropen) Substanzen, insbesondere Substanzen aus den Klassen der Azoxybenzole, Benzylidenaniline, Biphenyle, Terphenyle, Phenyl- oder Cyclohexylbenzoate, Cyclohexancarbonsäure-phenyl- oder cyclohexylester, Phenyl- oder Cyclohexylester der Cyclohexylbenzoesäure, Phenyl- oder Cyclohexylester der Cyclohexylcyclohexancarbonsäure, Cyclohexylphenylester der Benzoesäure, der Cyclohexancarbonsäure bzw. der Cyclohexylcyclohexancarbonsäure, Phenylcyclohexane, Cyclohexylbiphenyle, Phenylcyclohexylcyclohexane, Cyclohexylcyclohexane, Cyclohexylcyclohexylcyclohexene, 1,4-Bis-cyclohexylbenzole, 4,4'-Bis-cyclohexylbiphenyle, Phenyl- oder Cyclohexylpyrimidine, Phenyl- oder Cyclohexylpyridine, Phenyl- oder Cyclohexyldioxane, Phenyl- oder Cyclohexyl-1,3-dithiane, 1,2-Diphenylethane, 1,2-Dicyclohexylethane, 1-Phenyl-2-cyclohexylethane, 1-Cyclohexyl-2-(4-phenyl-cyclohexyl)-ethane, 1-Cyclohexyl-2-biphenylylethane, 1-Phenyl-2-cyclohexyl-phenylethane, gegebenenfalls halogenierten Stilbene, Benzylphenylether, Tolane und substituierten Zimtsäuren. Die 1,4-Phenylengruppen in diesen Verbindungen können auch fluoriert sein.

Die wichtigsten als weitere Bestandteile der erfindungsgemäßen Medien in Frage kommenden Verbindungen lassen sich durch die Formeln 1, 2, 3, 4 und 5 charakterisieren:

R'-L-E-R" 1

R'-L-COO-E-R" 2

R'-L-OOC-E-R" 3

R'-L-CH₂CH₂-E-R'' 4

R'-L-C≡C-E-R'' 5

In den Formeln 1, 2, 3, 4 und 5 bedeuten L und E, die gleich oder verschieden sein können, jeweils unabhängig voneinander einen bivalenten Rest aus der aus -Phe-, -Cyc-, -Phe-Phe-, -Phe-Cyc-, -Cyc-Cyc-, -Pyr-, -Dio-, -G-Phe- und -G-Cyc- sowie deren Spiegelbildern gebildeten Gruppe, wobei Phe unsubstituiertes oder durch Fluor substituiertes 1,4-Phenylen, Cyc trans-1,4-Cyclohexylen oder 1,4-Cyclohexenylen, Pyr Pyrimidin-2,5-diyl oder Pyridin-2,5-diyl, Dio 1,3-Dioxan-2,5-diyl und G 2-(trans-1,4-Cyclohexyl)-ethyl bedeuten.

Vorzugsweise ist einer der Reste L und E Cyc, Phe oder Pyr. E ist vorzugsweise Cyc, Phe oder Phe-Cyc. Vorzugsweise enthalten die erfindungsgemäßen Medien eine oder mehrere Komponenten ausgewählt aus den Verbindungen der Formeln 1, 2, 3, 4 und 5, worin L und E ausgewählt sind aus der Gruppe Cyc, Phe und Pyr und gleichzeitig eine oder mehrere Komponenten ausgewählt aus den Verbindungen der Formeln 1, 2, 3, 4 und 5, worin einer der Reste L und E ausgewählt ist aus der Gruppe Cyc, Phe und Pyr und der andere Rest ausgewählt ist aus der Gruppe -Phe-Phe-, -Phe-Cyc-, -Cyc-Cyc-, -G-Phe- und -G-Cyc-, und gegebenenfalls eine oder mehrere Komponenten ausgewählt aus den Verbindungen der Formeln 1, 2, 3, 4 und 5, worin die Reste L und E ausgewählt sind aus der Gruppe -Phe-Cyc-, -Cyc-Cyc-, -G-Phe- und -G-Cyc-.

R' und/oder R" bedeuten jeweils unabhängig voneinander Alkyl, Alkenyl, Alkoxy, Alkoxyalkyl, Alkenyloxy oder Alkanoyloxy mit bis zu 8 C-Atomen, -F, -Cl, -CN, -NCS, -(O)ᵢCH₃₋₍ₖ₊ₗ₎FₖClₗ, wobei i 0 oder 1, k und I unabhängig voneinander, gleich oder verschieden 0, 1, 2 oder 3 sind und für die Summe (k + I) gilt: 1 ≤ (k + I) ≤ 3.

R' und R" bedeuten in einer kleineren Untergruppe der Verbindungen der Formeln 1, 2, 3, 4 und 5 jeweils unabhängig voneinander Alkyl, Alkenyl, Alkoxy, Alkoxyalkyl, Alkenyloxy oder Alkanoyloxy mit bis zu 8 C-Atomen. Im folgenden wird diese kleinere Untergruppe Gruppe A genannt und die Verbindungen werden mit den Teilformeln 1a, 2a, 3a, 4a und 5a bezeichnet. Bei den meisten dieser Verbindungen sind R' und R" voneinander verschieden, wobei einer dieser Reste meist Alkyl, Alkenyl, Alkoxy oder Alkoxyalkyl ist.

In einer anderen als Gruppe B bezeichneten kleineren Untergruppe der Verbindungen der Formeln 1, 2, 3, 4 und 5 bedeutet R" -F, -Cl, -NCS oder -(O)ᵢ CH₃₋₍ₖ₊ₗ₎ FₖClₗ, wobei i 0 oder 1, k und I unabhängig voneinander, gleich oder verschieden 0, 1, 2 oder 3 sind und für die Summe (k + l) gilt: 1 ≤ (k + l) ≤ 3. Die Verbindungen, in denen R" diese Bedeutung hat, werden mit den Teilformeln 1b, 2b, 3b, 4b und 5b bezeichnet. Besonders bevorzugt sind solche Verbindungen der Teilformeln 1 b, 2b, 3b, 4b und 5b, in denen R" die Bedeutung -F, -Cl, -NCS, -CF₃, -OCHF₂ oder -OCF₃ hat.

In den Verbindungen der Teilformeln 1 b, 2b, 3b, 4b und 5b hat R' die bei den Verbindungen der Teilformeln 1 a bis 5a angegebene Bedeutung und ist vorzugsweise Alkyl, Alkenyl, Alkoxy oder Alkoxyalkyl.

In einer weiteren kleineren Untergruppe der Verbindungen der Formeln 1, 2, 3, 4 und 5 bedeutet R" -CN. Diese Untergruppe wird im folgenden als Gruppe C bezeichnet und die Verbindungen dieser Untergruppe werden entsprechend mit Teilformeln 1c, 2c, 3c, 4c und 5c beschrieben. In den Verbindungen der Teilformeln 1c, 2c, 3c, 4c und 5c hat R' die bei den Verbindungen der Teilformeln 1a bis 5a angegebene Bedeutung und ist vorzugsweise Alkyl, Alkoxy oder Alkenyl.

Neben den bevorzugten Verbindungen der Gruppen A, B und C sind auch andere Verbindungen der Formeln 1, 2, 3, 4 und 5 mit anderen Varianten der vorgesehenen Substituenten gebräuchlich. Alle diese Substanzen sind nach literaturbekannten Methoden oder in Analogie dazu erhältlich.

Die erfindungsgemäßen Medien enthalten neben erfindungsgemäßen Verbindungen der Formel I vorzugsweise eine oder mehrere Verbindungen, welche ausgewählt werden aus den Gruppen A, B und/oder C. Die Massenanteile der Verbindungen aus diesen Gruppen an den erfindungsgemäßen Medien sind vorzugsweise:
- Gruppe A:: 0 bis 90 %, vorzugsweise 20 bis 90 %, besonders bevorzugt 30 bis 90 %;
- Gruppe B:: 0 bis 80 %, vorzugsweise 10 bis 80%, besonders bevorzugt 10 bis 65 %;
- Gruppe C:: 0 bis 80 %, vorzugsweise 5 bis 80 %, besonders bevorzugt 5 bis 50 %;
wobei die Summe der Massenanteile der in den jeweiligen erfindungsgemäßen Medien enthaltenen Verbindungen aus den Gruppen A, B und/oder C vorzugsweise 5 bis 90 % und besonders bevorzugt 10 bis 90 % beträgt.

Die erfindungsgemäßen Medien enthalten vorzugsweise 1 bis 40 %, besonders bevorzugt 5 bis 30 % der erfindungsgemäßen Verbindungen der Formel I. Desweiteren bevorzugt sind Medien, enthaltend mehr als 40 %, besonders bevorzugt 45 bis 90 % an erfindungsgemäßen Verbindungen der Formel I. Die Medien enthalten vorzugsweise drei, vier oder fünf erfindungsgemäße Verbindungen.

Die Herstellung der erfindungsgemäßen Flüssigkristallmischungen erfolgt in an sich üblicher Weise. In der Regel wird die gewünschte Menge der in geringerer Menge verwendeten Komponenten in der den Hauptbestandteil ausmachenden Komponenten gelöst, vorzugsweise bei erhöhter Temperatur. Es ist auch möglich, Lösungen der Komponenten in einem organischen Lösungsmittel, z. B. in Aceton, Chloroform oder Methanol, zu mischen und das Lösungsmittel nach Durchmischung wieder zu entfernen, beispielsweise durch Destillation. Weiterhin ist es möglich, die Mischungen auf andere herkömmliche Arten, z. B. durch Verwendung von Vormischungen, z. B. Homologen-Mischungen oder unter Verwendung von sogenannten "Multi-Bottle"-Systemen herzustellen.

Die Dielektrika können auch weitere, dem Fachmann bekannte und in der Literatur beschriebene Zusätze enthalten. Beispielsweise können 0 bis 15 %, vorzugsweise 0 bis 10 %, pleochroitische Farbstoffe und/oder chirale Dotierstoffe zugesetzt werden. Die einzelnen, zugesetzten Verbindungen werden in Konzentrationen von 0,01 bis 6 %, vorzugsweise von 0,1 bis 3 %, eingesetzt. Dabei werden jedoch die Konzentrationsangaben der übrigen Bestandteile der Flüssigkristallmischungen also der flüssigkristallinen oder mesogenen Verbindungen, ohne Berücksichtigung der Konzentration dieser Zusatzstoffe angegeben.

In der vorliegenden Anmeldung und in den folgenden Beispielen sind die Strukturen der Flüssigkristallverbindungen durch Acronyme angegeben, wobei die Transformation in chemische Formeln gemäß der folgenden Tabellen A und B erfolgt. Alle Reste CₙH₂ₙ₊₁ und CₘH₂ₘ₊₁ sind geradkettige Alkylreste mit n bzw. m C-Atomen. n und m bedeuten ganze Zahlen, vorzugsweise 1, 2, 3, 4, 5, 6, 7, 8, 9, 10, 11 und 12, wobei n = m oder n ≠ m sein kann. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R^{1*}, R^{2*}, L^{1*} und L^{2*}:

| Code für R^{1*}, R^{2*}, L^{1*}, L^{2*} | R^{1*} | R^{2*} | L^{1*} | L^{2*} |
|---|---|---|---|---|
| nm | CₙH₂ₙ₊₁ | CₘH₂ₘ₊₁ | H | H |
| nOm | CₙH₂ₙ₊₁ | OCₘH₂ₘ₊₁ | H | H |
| nO.m | OCₙH₂ₙ₊₁ | CₘH₂ₘ₊₁ | H | H |
| nN | CₙH₂ₙ₊₁ | CN | H | H |
| nN.F | CₙH₂ₙ₊₁ | CN | H | F |
| nN.F.F | CₙH₂ₙ₊₁ | CN | F | F |
| nF | CₙH₂ₙ₊₁ | F | H | H |
| nOF | OCₙH₂ₙ₊₁ | F | H | H |
| nF.F | CₙH₂ₙ₊₁ | F | H | F |
| nmF | CₙH₂ₙ₊₁ | CₘH₂ₘ₊₁ | H | F |
| nCF₃ | CₙH₂ₙ₊₁ | CF₃ | H | H |
| nOCF₃ | CₙH₂ₙ₊₁ | OCF₃ | H | H |
| n-Vm | CₙH₂ₙ₊₁ | -CH=CH-CₘH₂ₘ₊₁ | H | H |
| nV-Vm | CₙH₂ₙ₊₁-CH=CH- | -CH=CH-CₘH₂ₘ₊₁ | H | H |

Bevorzugte Mischungskomponenten finden sich in den Tabellen A und B.

**Tabelle A:**

| | |
|---|---|
| 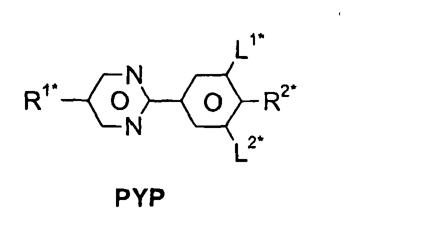 | 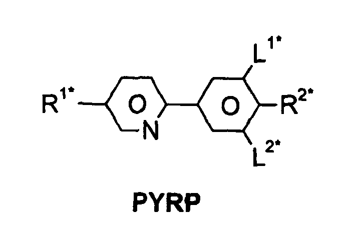 |
| 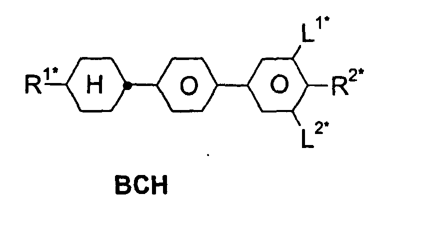 | 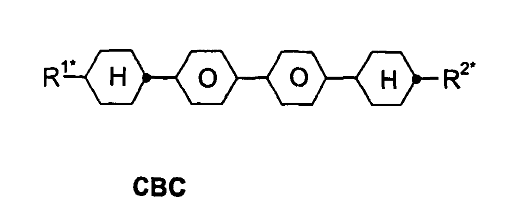 |
| 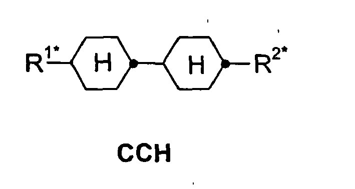 | 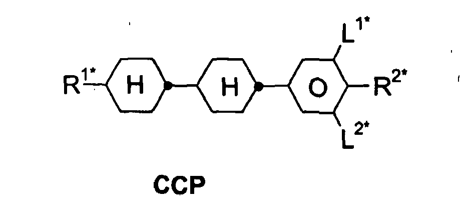 |
| 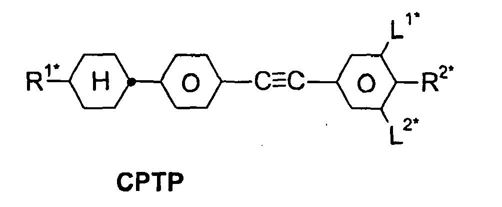 | |
| 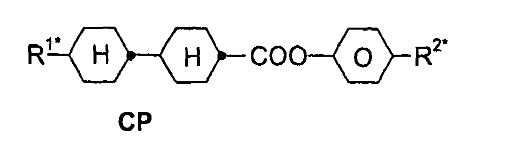 | |
| 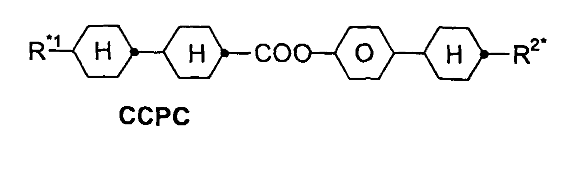 | |
| 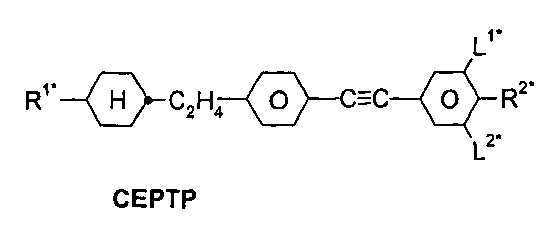 | |
| 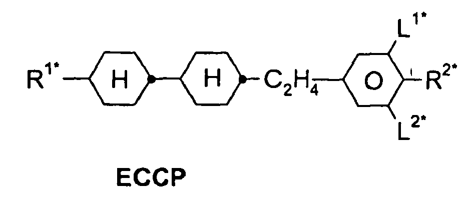 | 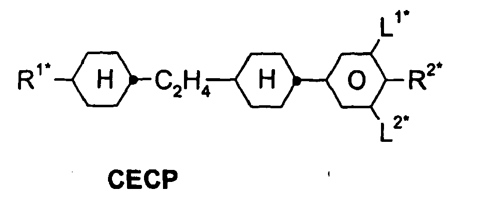 |
| 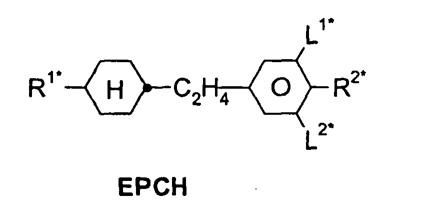 | 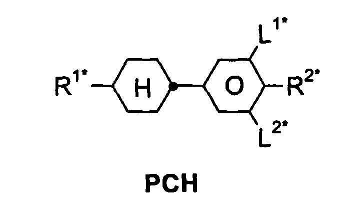 |
| 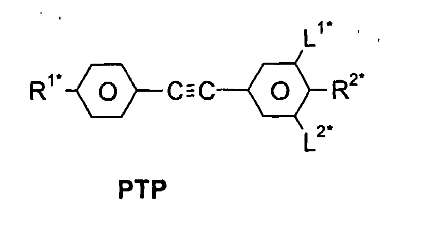 | 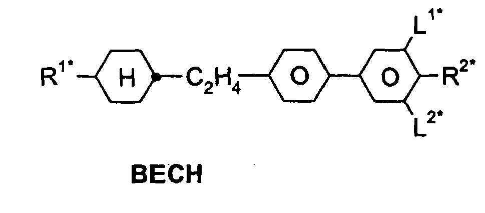 |
| 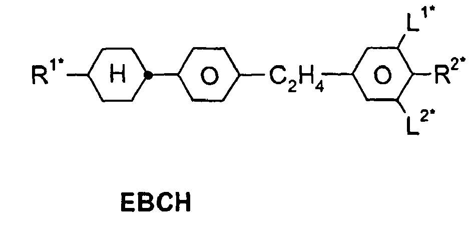 | 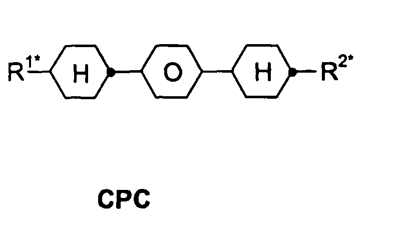 |
| 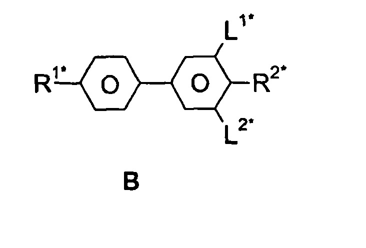 | 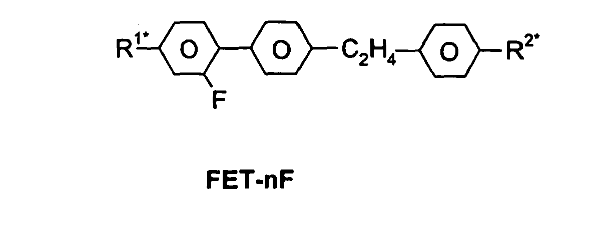 |
| 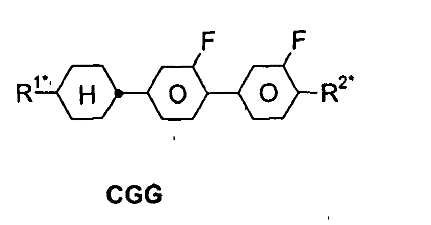 | 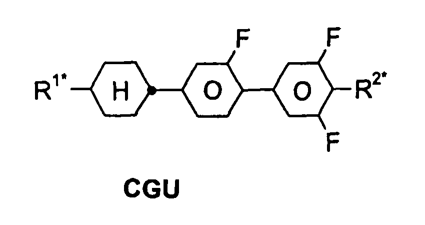 |
| 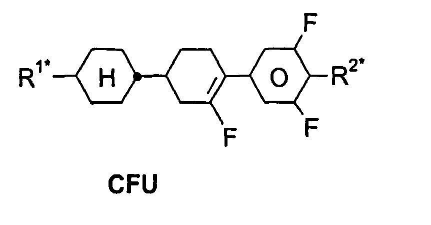 | |

**Tabelle B:**

| | |
|---|---|
| 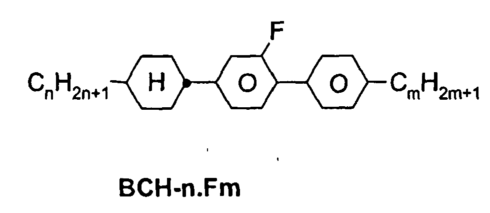 | 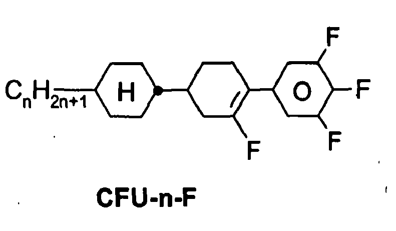 |
| 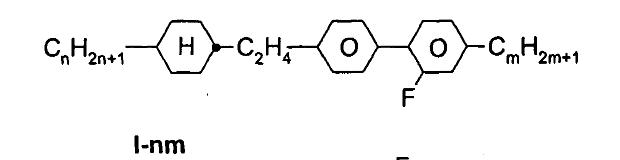 | |
| 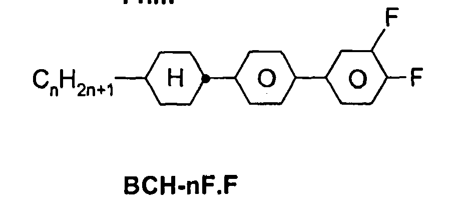 | 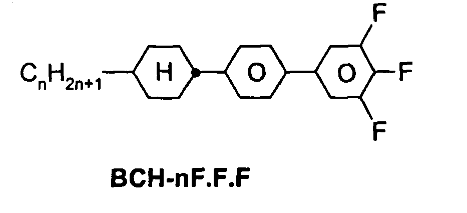 |
| 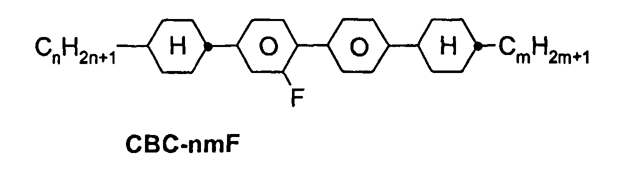 | |
| 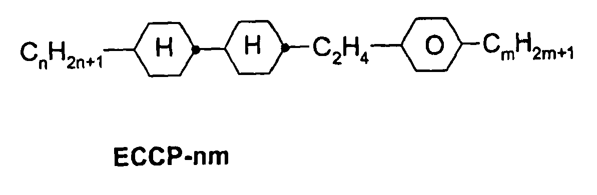 | |
| 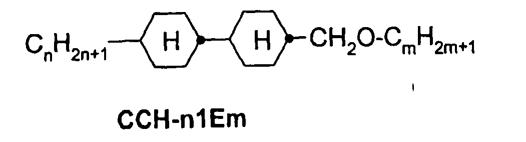 | |
| 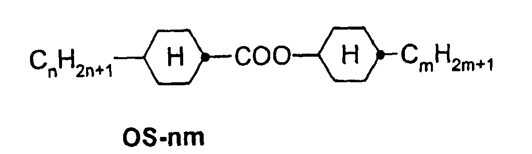 | |
| 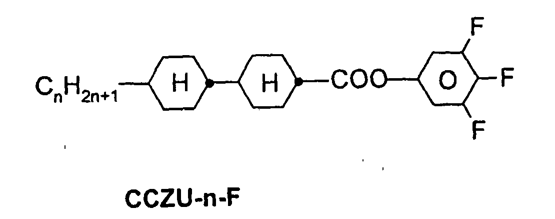 | |
| 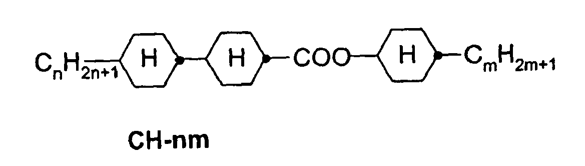 | 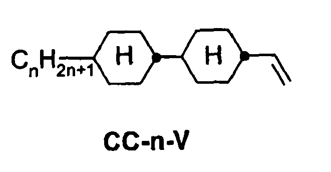 |
| 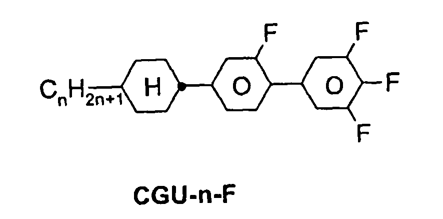 | 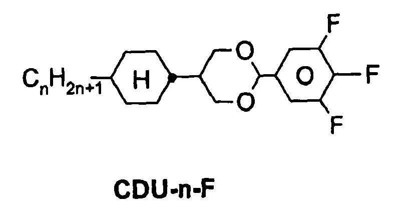 |
| 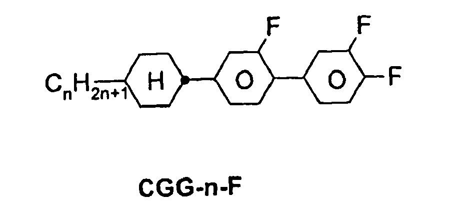 | 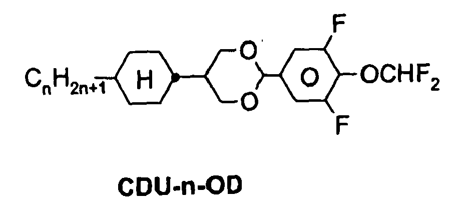 |
| 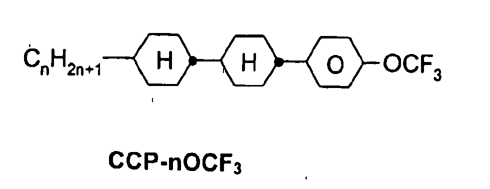 | 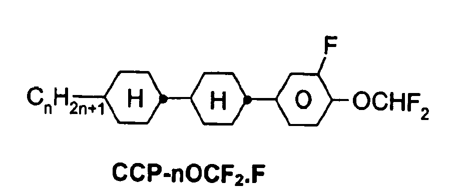 |
| 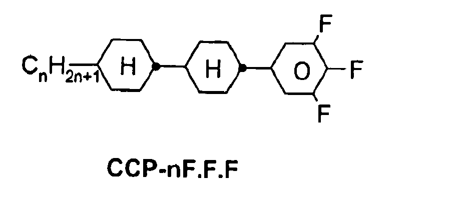 | 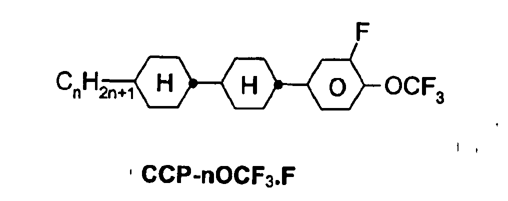 |
| 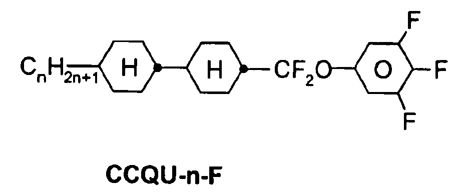 | 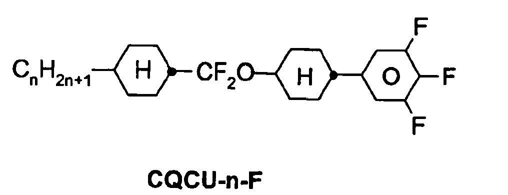 |
| 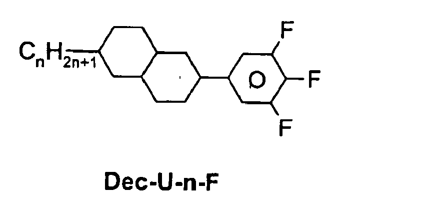 | 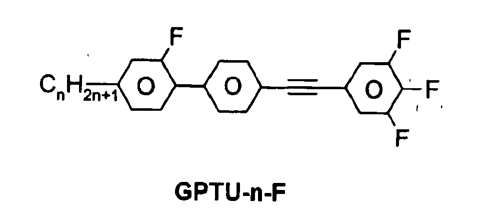 |
| 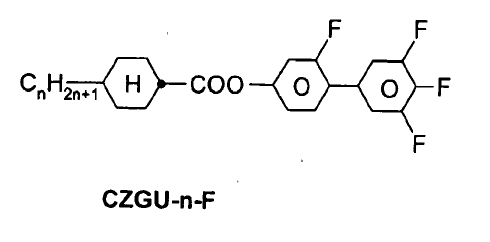 | 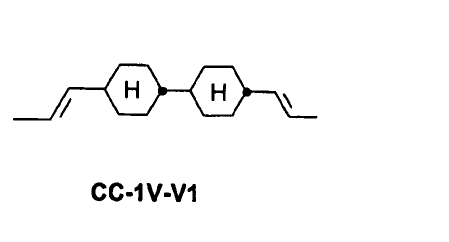 |
| 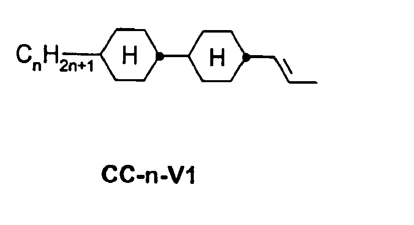 | 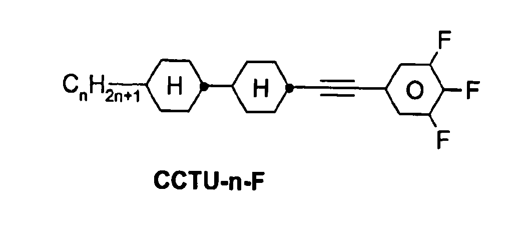 |
| 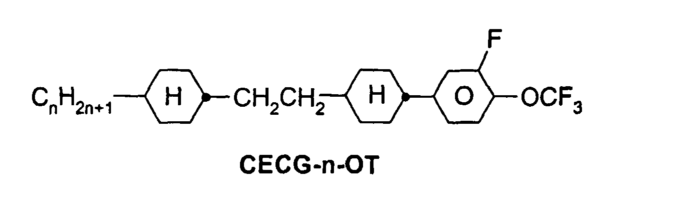 | |
| 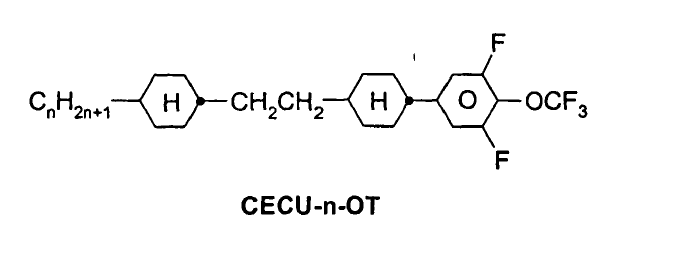 | |
| 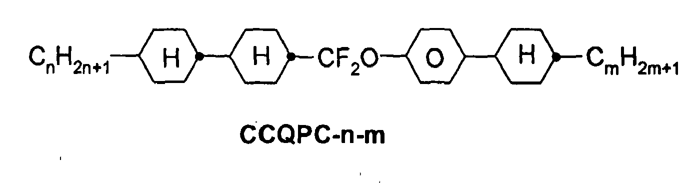 | |

**Tabelle C:**

| | |
|---|---|
| In der Tabelle C werden mögliche Dotierstoffe angegeben, die vorzugsweise in den erfindungsgemäßen Mischungen zugesetzt werden. | |
| 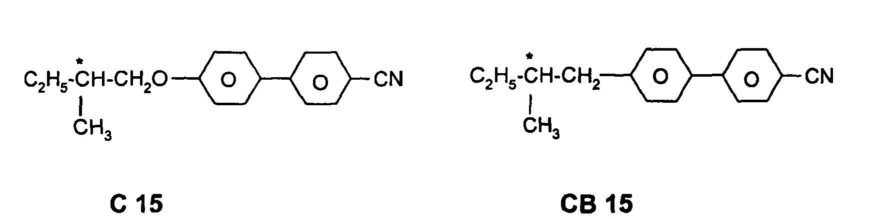 | 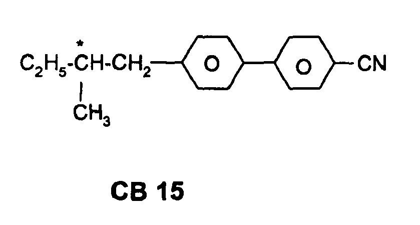 |
| 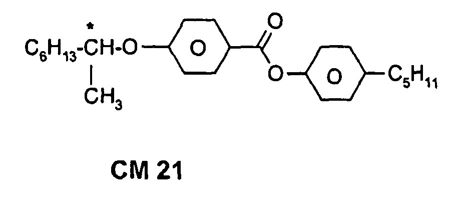 | |
| 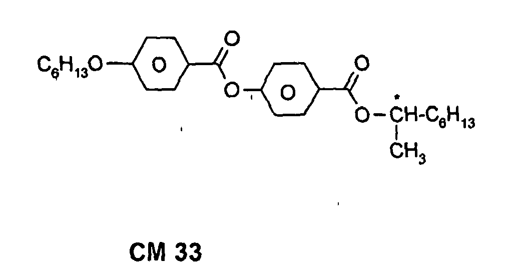 | |
| 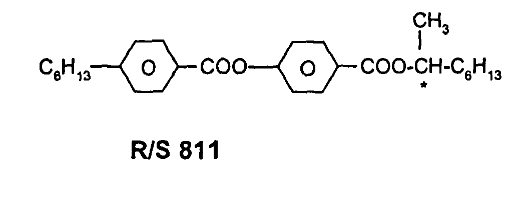 | |
| 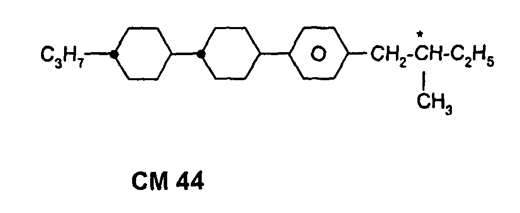 | |
| 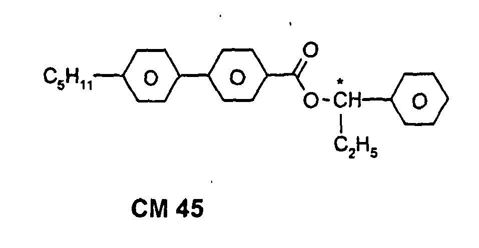 | 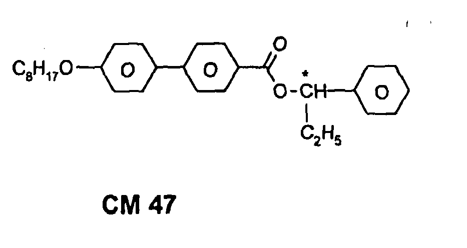 |
| 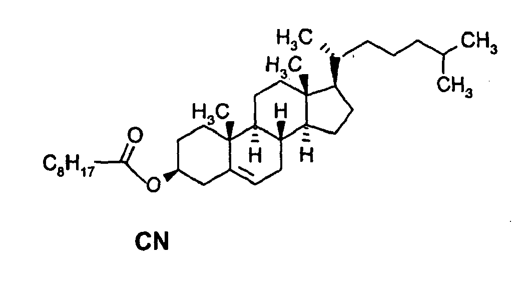 | |
| 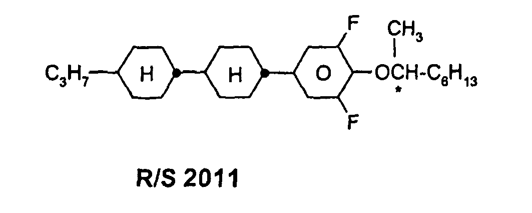 | |

Besonders bevorzugt sind erfindungsgemäße Mischungen, die neben einer oder mehreren Verbindungen der Formel I zwei, drei oder mehr Verbindungen ausgewählt aus der Tabelle B enthalten.

Die folgenden Beispiele sollen die Erfindung erläutern, ohne sie zu beschränken. Vor- und nachstehend bedeuten Prozentangaben Gewichtsprozent. Alle Temperaturen sind in Grad Celsius angegeben. Fp. bedeutet Schmelzpunkt und Klp. = Klärpunkt. Ferner bedeuten K = kristalliner Zustand, N = nematische Phase, Sm = smektische Phase und I = isotrope Phase. Die Angaben zwischen diesen Symbolen stellen die Übergangstemperaturen dar. Δn bedeutet optische Anisotropie (589 nm, 20°C) und Δε die dielektrische Anisotropie (1 kHz, 20°C).

Die Δn- und Δε-Werte der erfindungsgemäßen Verbindungen wurden durch Extrapolation aus flüssigkristallinen Mischungen erhalten, die zu 10 % aus der jeweiligen erfindungsgemäßen Verbindung und zu 90 % aus dem kommerziell erhältlichen Flüssigkristall ZLI 4792 (Fa. Merck, Darmstadt) bestanden.

"Übliche Aufarbeitung" bedeutet: man gibt gegebenenfalls Wasser hinzu, extrahiert mit Methylenchlorid, Diethylether oder Toluol, trennt ab, trocknet die organische Phase, dampft ein und reinigt das Produkt durch Destillation unter reduziertem Druck oder Kristallisation und/oder Chromatographie.

Vor- und nachstehend werden folgende Abkürzungen verwendet:
- DBH: 1,3-Dibrom-5,5-dimethylhydantoin
- DMF: Dimethylformamid
- LDA: Lithiumdiisopropylamid
- n-BuLi: n-Butyllithium
- RT: Raumtemperatur (etwa 20°C)
- THF: Tetrahydrofuran

### Beispiel 1

Die Verbindung der folgenden Formel

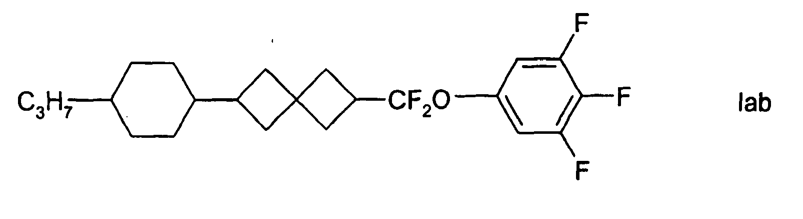

### 2-(Difluor-[3,4,5-trifluorphenyl]oxymethyl)-6-(4-propylcyclohexyl)spiro[3.3]-heptan [7]

wird wie folgt hergestellt:

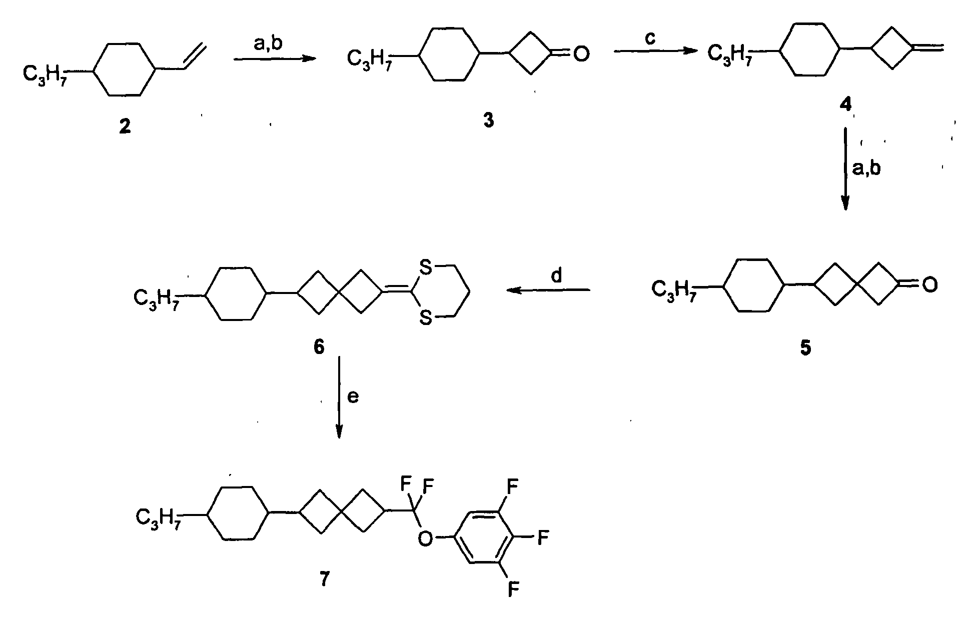

Reagenzien und Reaktionsbedingungen: a) Trichloracetylchlorid, Zn, Ether; b) Zn, HOAc; c) (Ph₃P)CH⁺Br⁻, KOtBu, THF; 2-Trimethylsilyl-1,3-dithian, nBuLi, THF, -78°C → RT; e) 1. 3,4,5-Trifluorphenol, Et₃N, CH₂Cl₂, -78°C; 2. Et₃N·3HF, DBH, 78°C → RT.

### 1. Stufe

### 2-(6-{4-n-Propylcyclohexyl}spiro[3.3]hept-2-yliden)[1,3]dithian 6

15,5 g (80,3 mmol) 2-Trimethylsilyl-1,3-dithian werden in 150 ml THF gelöst und bei -70°C 51 ml (80,3 mmol) n-BuLi (15 proz. in Hexan) zügig zutropfen gelassen. Anschließend läßt man den Ansatz innerhalb von **4** Stunden auf 0°C auftauen, rührt 10 Minuten ohne Kühlung, kühlt erneut auf -70°C ab und gibt eine Lösung von 17,0 g (72,5 mmol) 6-(4-n-Propylcyclohexyl)-spiro[3.3]heptan-2-on **5** in 50 ml THF tropfenweise hinzu. Der Ansatz wird über Nacht bei Raumtemperatur rühren gelassen, mit gesättigter Natriumhydrogencarbonatlösung hydrolysiert und mit Dichlormethan extrahiert. Die vereinigten organischen Phasen werden mit Wasser gewaschen und über Natriumsulfat getrocknet. Das Lösungsmittel wird im Vakuum entfernt und das Rohprodukt aus n-Heptan umkristallisiert. Man erhält das Dithioacetal **6** als farblose Blättchen.

### 2. Stufe

10,0 g (29,7 mmol) des Dithioacetals **6** werden in 100 ml Dichlormethan gelöst und unter Eis-Kochsalzkühlung 2,6 ml (30,0 mmol) Trifluormethansulfonsäure zutropfen gelassen. Nach 5 Minuten wird die Kühlung entfernt und der Ansatz 45 Minuten bei Raumtemperatur gerührt. Anschließend wird auf -70°C gekühlt, eine Mischung aus 7,5 ml (54,0 mmol) Triethylamin und 6,67 g (45,0 mol) Trifluorphenol in 30 ml Dichlormethan hinzugegeben und 1 Stunde bei -70°C gerührt. Dann werden 24,2 ml (0,150 mol) Triethylamin-tris(hydrofluorid) hinzugegeben und nach 5 Minuten 42,9 g (0,150 mol) DBH, suspendiert in 60 ml Dichlormethan, portionsweise innerhalb von ca. 30 Minuten hinzugegeben. Nach 90 Minuten wird der Ansatz auftauen gelassen und mit 1 M Natronlauge und wäßriger Hydrogensulfitlösung hydrolysiert. Die organische Phase wird abgetrennt, mit gesättigter Natriumchloridlösung gewaschen und über Natriumsulfat getrocknet. Das Lösungsmittel wird im Vakuum entfernt und der Rückstand mit n-Heptan über Kieselgel filtriert. Man erhält die Spiroverbindung **7** als farblosen Feststoff vom Schmelzpunkt: 43°C.
K 43 N 46,6 I
Δε = 9,1
Δn = 0,0668
γ₁ = 132 mPa·s
¹⁹F-NMR (235 MHz, CDCl₃)
δ = -79,2 ppm (d, ³J_{F,H} = 10,6 Hz, 2 F, CF₂O), -133,2 (m_{c}, 2 F, Ar-F), -164,7 (tt, ³J_{F,F} = 20,8 Hz, ⁴J_{F,H} = 5,9 Hz, 1 F, Ar-F).

Analog Beispiel 1 werden unter Verwendung der entsprechenden Vorstufen die folgenden erfindungsgemäßen Verbindungen erhalten:

### Beispiele 2-108

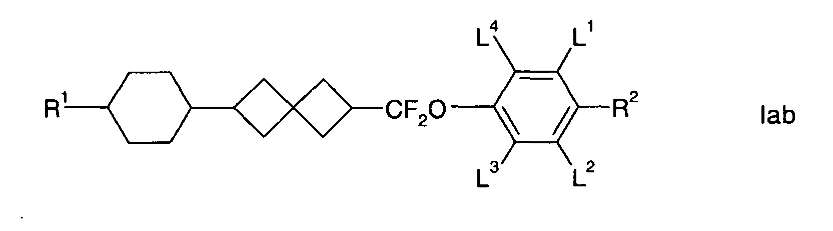

### Beispiele 109-216

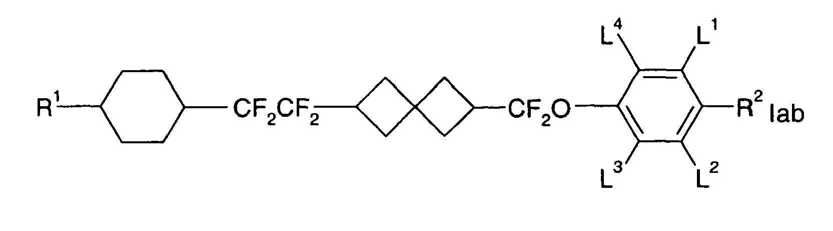

### Beispiele 217-324

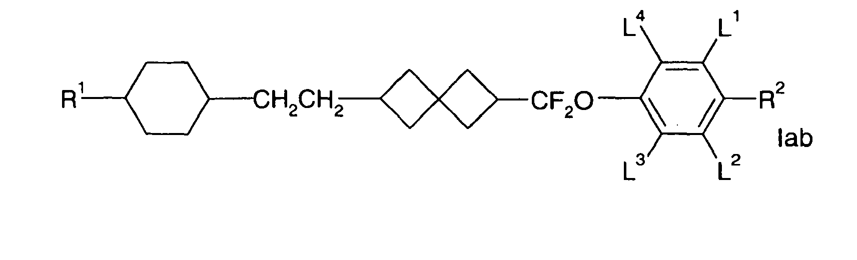

### Beispiele 325-432

| | | | | | | | | | |
|---|---|---|---|---|---|---|---|---|---|
| 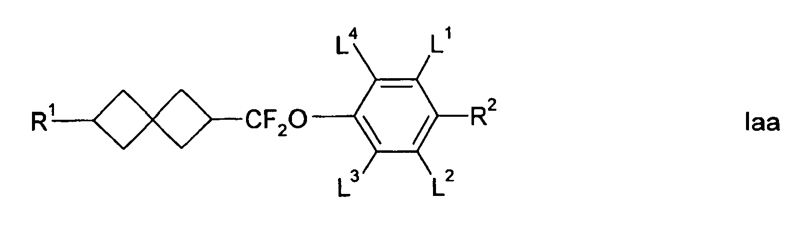 | | | | | | | | | |

| **Beispiele** | | | | **R¹** | **L¹** | **L²** | **L³** | **L⁴** | **R²** |
|---|---|---|---|---|---|---|---|---|---|
| 1. | 109. | 217. | 325. | C₃H₇ | F | F | H | H | F |
| 2. | 110. | 218. | 326. | C₃H₇ | F | H | H | H | F |
| 3. | 111. | 219. | 327. | C₃H₇ | H | H | H | H | F |
| 4. | 112. | 220. | 328. | C₂H₅ | H | H | H | H | F |
| 5. | 113. | 221. | 329. | C₂H₅ | F | H | H | H | F |
| 6. | 114. | 222. | 330. | C₂H₅ | F | F | H | H | F |
| 7. | 115. | 223. | 331. | C₄H₉ | H | H | H | H | F |
| 8. | 116. | 224. | 332. | C₄H₉ | F | H | H | H | F |
| 9. | 117. | 225. | 333. | C₄H₉ | F | F | H | H | F |
| 10. | 118. | 226. | 334. | C₅H₁₁ | H | H | H | H | F |
| 11. | 119. | 227. | 335. | C₅H₁₁ | F | H | H | H | F |
| 12. | 120. | 228. | 336. | C₅H₁₁ | F | F | H | H | F |
| 13. | 121. | 229. | 337. | C₆H₁₃ | H | H | H | H | F |
| 14. | 122. | 230. | 338. | C₆H₁₃ | F | H | H | H | F |
| 15. | 123. | 231. | 339. | C₆H₁₃ | F | F | H | H | F |
| 16. | 124. | 232. | 340. | C₇H₁₅ | H | H | H | H | F |
| 17. | 125. | 233. | 341. | C₇H₁₅ | F | H | H | H | F |
| 18. | 126. | 234. | 342. | C₇H₁₅ | F | F | H | H | F |
| 19. | 127. | 235. | 343. | C₂H₅ | H | H | H | H | CF₃ |
| 20. | 128. | 236. | 344. | C₂H₅ | F | H | H | H | CF₃ |
| 21. | 129. | 237. | 345. | C₂H₅ | F | F | H | H | CF₃ |
| 22. | 130. | 238. | 346. | C₃H₇ | H | H | H | H | CF₃ |
| 23. | 131. | 239. | 347. | C₃H₇ | F | H | H | H | CF₃ |
| 24. | 132. | 240. | 348. | C₃H₇ | F | F | H | H | CF₃ |
| 25. | 133. | 241. | 349. | C₄H₉ | H | H | H | H | CF₃ |
| 26. | 134. | 242. | 350. | C₄H₉ | F | H | H | H | CF₃ |
| 27. | 135. | 243. | 351. | C₄H₉ | F | F | H | H | CF₃ |
| 28. | 136. | 244. | 352. | C₅H₁₁ | H | H | H | H | CF₃ |
| 29. | 137. | 245. | 353. | C₅H₁₁ | F | H | H | H | CF₃ |
| 30. | 138. | 246. | 354. | C₅H₁₁ | F | F | H | H | CF₃ |
| 31. | 139. | 247. | 355. | C₆H₁₃ | H | H | H | H | CF₃ |
| 32. | 140. | 248. | 356. | C₆H₁₃ | F | H | H | H | CF₃ |
| 33. | 141. | 249. | 357. | C₆H₁₃ | F | F | H | H | CF₃ |
| 34. | 142. | 250. | 358. | C₇H₁₅ | H | H | H | H | CF₃ |
| 35. | 143. | 251. | 359. | C₇H₁₅ | F | H | H | H | CF₃ |
| 36. | 144. | 252. | 360. | C₇H₁₅ | F | F | H | H | CF₃ |
| 37. | 145. | 253. | 361. | C₂H₅ | H | H | H | H | OCF₃ |
| 38. | 146. | 254. | 362. | C₂H₅ | F | H | H | H | OCF₃ |
| 39. | 147. | 255. | 363. | C₂H₅ | F | F | H | H | OCF₃ |
| 40. | 148. | 256. | 364. | C₃H₇ | H | H | H | H | OCF₃ |
| 41. | 149. | 257. | 365. | C₃H₇ | F | H | H | H | OCF₃ |
| 42. | 150. | 258. | 366. | C₃H₇ | F | F | H | H | OCF₃ |
| 43. | 151. | 259. | 367. | C₄H₉ | H | H | H | H | OCF3 |
| 44. | 152. | 260. | 368. | C₄H₉ | F | H | H | H | OCF₃ |
| 45. | 153. | 261. | 369. | C₄H₉ | F | F | H | H | OCF₃ |
| 46. | 154. | 262. | 370. | C₅H₁₁ | H | H | H | H | OCF₃ |
| 47. | 155. | 263. | 371. | C₅H₁₁ | F | H | H | H | OCF₃ |
| 48. | 156. | 264. | 372. | C₅H₁₁ | F | F | H | H | OCF₃ |
| 49. | 157. | 265. | 373. | C₆H₁₃ | H | H | H | H | OCF₃ |
| 50. | 158. | 266. | 374. | C₆H₁₃ | F | H | H | H | OCF₃ |
| 51. | 159. | 267. | 375. | C₆H₁₃ | F | F | H | H | OCF₃ |
| 52. | 160. | 268. | 376. | C₇H₁₅ | H | H | H | H | OCF₃ |
| 53. | 161. | 269. | 377. | C₇H₁₅ | F | H | H | H | OCF₃ |
| 54. | 162. | 270. | 378. | C₇H₁₅ | F | F | H | H | OCF₃ |
| 55. | 163. | 271. | 379. | C₂H₅ | H | H | H | H | CN |
| 56. | 164. | 272. | 380. | C₂H₅ | F | H | H | H | CN |
| 57. | 165. | 273. | 381. | C₂H₅ | F | F | H | H | CN |
| 58. | 166. | 274. | 382. | C₃H₇ | H | H | H | H | CN |
| 59. | 167. | 275. | 383. | C₃H₇ | F | H | H | H | CN |
| 60. | 168. | 276. | 384. | C₃H₇ | F | F | H | H | CN |
| 61. | 169. | 277. | 385. | C₄H₉ | H | H | H | H | CN |
| 62. | 170. | 278. | 386. | C₄H₉ | F | H | H | H | CN |
| 63. | 171. | 279. | 387. | C₄H₉ | F | F | H | H | CN |
| 64. | 172. | 280. | 388. | C₅H₁₁ | H | H | H | H | CN |
| 65. | 173. | 281. | 389. | C₅H₁₁ | F | H | H | H | CN |
| 66. | 174. | 282. | 390. | C₅H₁₁ | F | F | H | H | CN |
| 67. | 175. | 283. | 391. | C₆H₁₃ | H | H | H | H | CN |
| 68. | 176. | 284. | 392. | C₆H₁₃ | F | H | H | H | CN |
| 69. | 177. | 285. | 393. | C₆H₁₃ | F | F | H | H | CN |
| 70. | 178. | 286. | 394. | C₇H₁₅ | H | H | H | H | CN |
| 71. | 179. | 287. | 395. | C₇H₁₅ | F | H | H | H | CN |
| 72. | 180. | 288. | 396. | C₇H₁₅ | F | F | H | H | CN |
| 73. | 181. | 289. | 397. | C₂H₅ | H | H | H | H | SF₅ |
| 74. | 182. | 290. | 398. | C₂H₅ | F | H | H | H | SF₅ |
| 75. | 183. | 291. | 399. | C₂H₅ | F | F | H | H | SF₅ |
| 76. | 184. | 292. | 400. | C₃H₇ | H | H | H | H | SF₅ |
| 77. | 185. | 293. | 401. | C₃H₇ | F | H | H | H | SF₅ |
| 78. | 186. | 294. | 402. | C₃H₇ | F | F | H | H | SF₅ |
| 79. | 187. | 295. | 403. | C₄H₉ | H | H | H | H | SF₅ |
| 80. | 188. | 296. | 404. | C₄H₉ | F | H | H | H | SF₅ |
| 81. | 189. | 297. | 405. | C₄H₉ | F | F | H | H | SF₅ |
| 82. | 190. | 298. | 406. | C₅H₁₁ | H | H | H | H | SF₅ |
| 83. | 191. | 299. | 407. | C₅H₁₁ | F | H | H | H | SF₅ |
| 84. | 192. | 300. | 408. | C₅H₁₁ | F | F | H | H | SF₅ |
| 85. | 193. | 301. | 409. | C₆H₁₃ | H | H | H | H | SF₅ |
| 86. | 194. | 302. | 410. | C₆H₁₃ | F | H | H | H | SF₅ |
| 87. | 195. | 303. | 411. | C₆H₁₃ | F | F | H | H | SF₅ |
| 88. | 196. | 304. | 412. | C₇H₁₅ | H | H | H | H | SF₅ |
| 89. | 197. | 305. | 413. | C₇H₁₅ | F | H | H | H | SF₅ |
| 90. | 198. | 306. | 414. | C₇H₁₅ | F | F | H | H | SF₅ |
| 91. | 199. | 307. | 415. | C₂H₅ | H | H | H | H | OC₂H₅ |
| 92. | 200. | 308. | 416. | C₂H₅ | F | H | H | H | OC₂H₅ |
| 93. | 201. | 309. | 417. | C₂H₅ | F | H | F | H | OC₂H₅ |
| 94. | 202. | 310. | 418. | C₃H₇ | H | H | H | H | OC₂H₅ |
| 95. | 203. | 311. | 419. | C₃H₇ | F | H | H | H | OC₂H₅ |
| 96. | 204. | 312. | 420. | C₃H₇ | F | H | F | H | OC₂H₅ |
| 97. | 205. | 313. | 421. | C₄H₉ | H | H | H | H | OC₂H₅ |
| 98. | 206. | 314. | 422. | C₄H₉ | F | H | H | H | OC₂H₅ |
| 99. | 207. | 315. | 423. | C₄H₉ | F | H | F | H | OC₂H₅ |
| 100. | 208. | 316. | 424. | C₅H₁₁ | H | H | H | H | OC₂H₅ |
| 101. | 209. | 317. | 425. | C₅H₁₁ | F | H | H | H | OC₂H₅ |
| 102. | 210. | 318. | 426. | C₅H₁₁ | F | H | F | H | OC₂H₅ |
| 103. | 211. | 319. | 427. | C₆H₁₃ | H | H | H | H | OC₂H₅ |
| 104. | 212. | 320. | 428. | C₆H₁₃ | F | H | H | H | OC₂H₅ |
| 105. | 213. | 321. | 429. | C₆H₁₃ | F | H | F | H | OC₂H₅ |
| 106. | 214. | 322. | 430. | C₇H₁₅ | H | H | H | H | OC₂H₅ |
| 107. | 215. | 323. | 431. | C₇H₁₅ | F | H | H | H | OC₂H₅ |
| 108. | 216. | 324. | 432. | C₇H₁₅ | F | H | F | H | OC₂H₅ |

### Beispiel 433

Die Verbindung der folgenden Formel

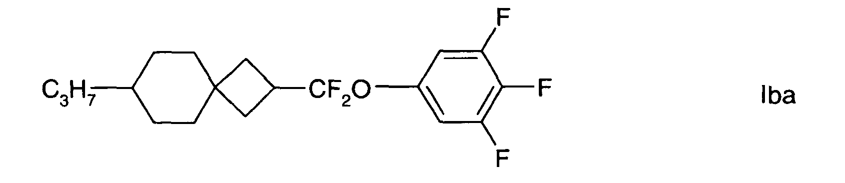

### 2-(Difluor-[3,4,5-trifluorphenyl]oxymethyl)-7-(4-n-propylcyclohexyl)-spiro[5.3]nonan (11)

wird wie folgt hergestellt:

In Analogie zu der in Beispiel 1 beschriebenen Synthese wird 2-(Difluor-[3,4,5-trifluorphenyl]oxymethyl)-7-(4-n-propylcyclohexyl)spiro[5.3]nonan (11) aus 7-(4-n-propylcyclohexyl)spiro[5.3]nonan-2-on (9) erhalten.

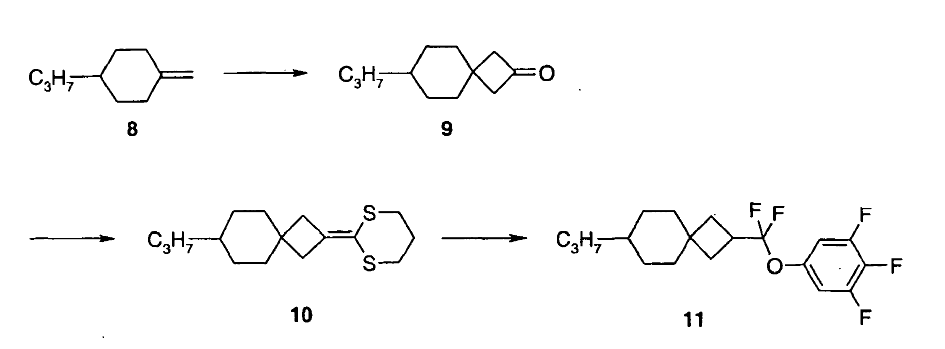

8,90 g (32,0 mmol) des Dithioacetals **10** werden in 80 ml Dichlormethan gelöst und unter Eis-Kochsalzkühlung 2,8 ml (32,0 mmol) Trifluormethansulfonsäure zutropfen gelassen. Nach 5 Minuten wird die Kühlung entfernt und der Ansatz 45 Minuten bei Raumtemperatur gerührt. Anschließend wird auf -70°C gekühlt, eine Mischung aus 7,1 ml (51,0 mmol) Triethylamin und 7,10 g (48,0 mmol) Trifluorphenol in 80 ml Dichlormethan hinzugegeben und 1 Stunde bei -70°C gerührt. Dann werden 25,8 ml (0,160 mol) Triethylamin-tris(hydrofluorid) hinzugegeben und nach 5 Minuten 45,8 g (0,150 mol) DBH, suspendiert in 60 ml Dichlormethan, portionsweise innerhalb von ca. 30 Minuten hinzugegeben. Nach 90 Minuten wird der Ansatz auftauen gelassen und mit 1 M Natronlauge und wäßriger Hydrogensulfitlösung hydrolysiert. Die organische Phase wird abgetrennt, mit gesättigter Natriumchloridlösung gewaschen und über Natriumsulfat getrocknet. Das Lösungsmittel wird im Vakuum entfernt und der Rückstand mit n-Heptan über Kieselgel filtriert. Das Rohprodukt wird durch Kugelrohrdestillation gereinigt (Siedepunkt: 158°C/0,1 mbar). Man erhält die Spiroverbindung **11**.
Δε = 7,4
Δn = 0,0641

Analog Beispiel 433 werden unter Verwendung der entsprechenden Vorstufen die folgenden, erfindungsgemäßen Verbindungen erhalten:

### Beispiele 434-540

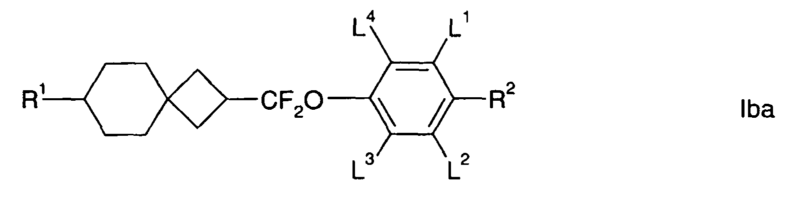

### Beispiele 541-648

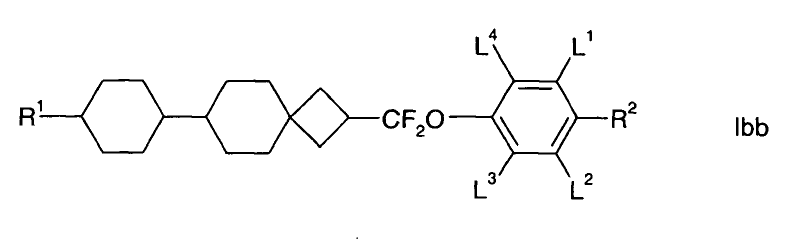

### Beispiele 649-756

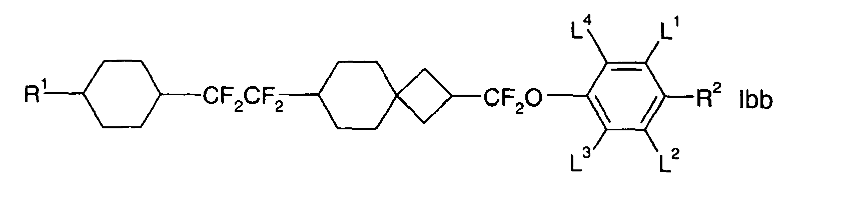

### Beispiele 757-864

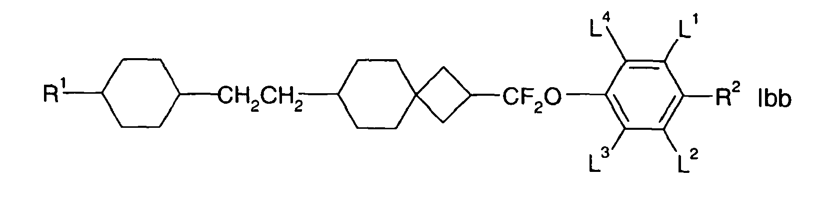

| **Beispiele** | | | | **R¹** | **L¹** | **L²** | **L³** | **L⁴** | **R²** |
|---|---|---|---|---|---|---|---|---|---|
| 433. | 541. | 649. | 757. | C₃H₇ | F | F | H | H | F |
| 434. | 542. | 650. | 758. | C₃H₇ | F | H | H | H | F |
| 435. | 543. | 651. | 759. | C₃H₇ | H | H | H | H | F |
| 436. | 544. | 652. | 760. | C₂H₅ | H | H | H | H | F |
| 437. | 545. | 653. | 761. | C₂H₅ | F | H | H | H | F |
| 438. | 546. | 654. | 762. | C₂H₅ | F | F | H | H | F |
| 439. | 547. | 655. | 763. | C₄H₉ | H | H | H | H | F |
| 440. | 548. | 656. | 764. | C₄H₉ | F | H | H | H | F |
| 441. | 549. | 657. | 765. | C₄H₉ | F | F | H | H | F |
| 442. | 550. | 658. | 766. | C₅H₁₁ | H | H | H | H | F |
| 443. | 551. | 659. | 767. | C₅H₁₁ | F | H | H | H | F |
| 444. | 552. | 660. | 768. | C₅H₁₁ | F | F | H | H | F |
| 445. | 553. | 661. | 769. | C₆H₁₃ | H | H | H | H | F |
| 446. | 554. | 662. | 770. | C₆H₁₃ | F | H | H | H | F |
| 447. | 555. | 663. | 771. | C₆H₁₃ | F | F | H | H | F |
| 448. | 556. | 664. | 772. | C₇H₁₅ | H | H | H | H | F |
| 449. | 557. | 665. | 773. | C₇H₁₅ | F | H | H | H | F |
| 450. | 558. | 666. | 774. | C₇H₁₅ | F | F | H | H | F |
| 451. | 559. | 667. | 775. | C₂H₅ | H | H | H | H | CF₃ |
| 452. | 560. | 668. | 776. | C₂H₅ | F | H | H | H | CF₃ |
| 453. | 561. | 669. | 777. | C₂H₅ | F | F | H | H | CF₃ |
| 454. | 562. | 670. | 778. | C₃H₇ | H | H | H | H | CF₃ |
| 455. | 563. | 671. | 779. | C₃H₇ | F | H | H | H | CF₃ |
| 456. | 564. | 672. | 780. | C₃H₇ | F | F | H | H | CF₃ |
| 457. | 565. | 673. | 781. | C₄H₉ | H | H | H | H | CF₃ |
| 458. | 566. | 674. | 782. | C₄H₉ | F | H | H | H | CF₃ |
| 459. | 567. | 675. | 783. | C₄H₉ | F | F | H | H | CF₃ |
| 460. | 568. | 676. | 784. | C₅H₁₁ | H | H | H | H | CF₃ |
| 461. | 569. | 677. | 785. | C₅H₁₁ | F | H | H | H | CF₃ |
| 462. | 570. | 678. | 786. | C₅H₁₁ | F | F | H | H | CF₃ |
| 463. | 571. | 679. | 787. | C₆H₁₃ | H | H | H | H | CF₃ |
| 464. | 572. | 680. | 788. | C₆H₁₃ | F | H | H | H | CF₃ |
| 465. | 573. | 681. | 789. | C₆H₁₃ | F | F | H | H | CF₃ |
| 466. | 574. | 682. | 790. | C₇H₁₅ | H | H | H | H | CF₃ |
| 467. | 575. | 683. | 791. | C₇H₁₅ | F | H | H | H | CF₃ |
| 468. | 576. | 684. | 792. | C₇H₁₅ | F | F | H | H | CF₃ |
| 469. | 577. | 685. | 793. | C₂H₅ | H | H | H | H | OCF₃ |
| 470. | 578. | 686. | 794. | C₂H₅ | F | H | H | H | OCF₃ |
| 471. | 579. | 687. | 795. | C₂H₅ | F | F | H | H | OCF3 |
| 472. | 580. | 688. | 796. | C₃H₇ | H | H | H | H | OCF₃ |
| 473. | 581. | 689. | 797. | C₃H₇ | F | H | H | H | OCF₃ |
| 474. | 582. | 690. | 798. | C₃H₇ | F | F | H | H | OCF₃ |
| 475. | 583. | 691. | 799. | C₄H₉ | H | H | H | H | OCF₃ |
| 476. | 584. | 692. | 800. | C₄H₉ | F | H | H | H | OCF₃ |
| 477. | 585. | 693. | 801. | C₄H₉ | F | F | H | H | OCF₃ |
| 478. | 586. | 694. | 802. | C₅H₁₁ | H | H | H | H | OCF₃ |
| 479. | 587. | 695. | 803. | C₅H₁₁ | F | H | H | H | OCF₃ |
| 480. | 588. | 696. | 804. | C₅H₁₁ | F | F | H | H | OCF₃ |
| 481. | 589. | 697. | 805. | C₆H₁₃ | H | H | H | H | OCF₃ |
| 482. | 590. | 698. | 806. | C₆H₁₃ | F | H | H | H | OCF₃ |
| 483. | 591. | 699. | 807. | C₆H₁₃ | F | F | H | H | OCF₃ |
| 484. | 592. | 700. | 808. | C₇H₁₅ | H | H | H | H | OCF₃ |
| 485. | 593. | 701. | 809. | C₇H₁₅ | F | H | H | H | OCF₃ |
| 486. | 594. | 702. | 810. | C₇H₁₅ | F | F | H | H | OCF₃ |
| 487. | 595. | 703. | 811. | C₂H₅ | H | H | H | H | CN |
| 488. | 596. | 704. | 812. | C₂H₅ | F | H | H | H | CN |
| 489. | 597. | 705. | 813. | C₂H₅ | F | F | H | H | CN |
| 490. | 598. | 706. | 814. | C₃H₇ | H | H | H | H | CN |
| 491. | 599. | 707. | 815. | C₃H₇ | F | H | H | H | CN |
| 492. | 600. | 708. | 816. | C₃H₇ | F | F | H | H | CN |
| 493. | 601. | 709. | 817. | C₄H₉ | H | H | H | H | CN |
| 494. | 602. | 710. | 818. | C₄H₉ | F | H | H | H | CN |
| 495. | 603. | 711. | 819. | C₄H₉ | F | F | H | H | CN |
| 496. | 604. | 712. | 820. | C₅H₁₁ | H | H | H | H | CN |
| 497. | 605. | 713. | 821. | C₅H₁₁ | F | H | H | H | CN |
| 498. | 606. | 714. | 822. | C₅H₁₁ | F | F | H | H | CN |
| 499. | 607. | 715. | 823. | C₆H₁₃ | H | H | H | H | CN |
| 500. | 608. | 716. | 824. | C₆H₁₃ | F | H | H | H | CN |
| 501. | 609. | 717. | 825. | C₆H₁₃ | F | F | H | H | CN |
| 502. | 610. | 718. | 826. | C₇H₁₅ | H | H | H | H | CN |
| 503. | 611. | 719. | 827. | C₇H₁₅ | F | H | H | H | CN |
| 504. | 612. | 720. | 828. | C₇H₁₅ | F | F | H | H | CN |
| 505. | 613. | 721. | 829. | C₂H₅ | H | H | H | H | SF₅ |
| 506. | 614. | 722. | 830. | C₂H₅ | F | H | H | H | SF₅ |
| 507. | 615. | 723. | 831. | C₂H₅ | F | F | H | H | SF₅ |
| 508. | 616. | 724. | 832. | C₃H₇ | H | H | H | H | SF₅ |
| 509. | 617. | 725. | 833. | C₃H₇ | F | H | H | H | SF₅ |
| 510. | 618. | 726. | 834. | C₃H₇ | F | F | H | H | SF₅ |
| 511. | 619. | 727. | 835. | C₄H₉ | H | H | H | H | SF₅ |
| 512. | 620. | 728. | 836. | C₄H₉ | F | H | H | H | SF₅ |
| 513. | 621. | 729. | 837. | C₄H₉ | F | F | H | H | SF₅ |
| 514. | 622. | 730. | 838. | C₅H₁₁ | H | H | H | H | SF₅ |
| 515. | 623. | 731. | 839. | C₅H₁₁ | F | H | H | H | SF₅ |
| 516. | 624. | 732. | 840. | C₅H₁₁ | F | F | H | H | SF₅ |
| 517. | 625. | 733. | 841. | C₆H₁₃ | H | H | H | H | SF₅ |
| 518. | 626. | 734. | 842. | C₆H₁₃ | F | H | H | H | SF₅ |
| 519. | 627. | 735. | 843. | C₆H₁₃ | F | F | H | H | SF₅ |
| 520. | 628. | 736. | 844. | C₇H₁₅ | H | H | H | H | SF₅ |
| 521. | 629. | 737. | 845. | C₇H₁₅ | F | H | H | H | SF₅ |
| 522. | 630. | 738. | 846. | C₇H₁₅ | F | F | H | H | SF₅ |
| 523. | 631. | 739. | 847. | C₂H₅ | H | H | H | H | OC₂H₅ |
| 524. | 632. | 740. | 848. | C₂H₅ | F | H | H | H | OC₂H₅ |
| 525. | 633. | 741. | 849. | C₂H₅ | F | H | F | H | OC₂H₅ |
| 526. | 634. | 742. | 850. | C₃H₇ | H | H | H | H | OC₂H₅ |
| 527. | 635. | 743. | 851. | C₃H₇ | F | H | H | H | OC₂H₅ |
| 528. | 636. | 744. | 852. | C₃H₇ | F | H | F | H | OC₂H₅ |
| 529. | 637. | 745. | 853. | C₄H₉ | H | H | H | H | OC₂H₅ |
| 530. | 638. | 746. | 854. | C₄H₉ | F | H | H | H | OC₂H₅ |
| 531. | 639. | 747. | 855. | C₄H₉ | F | H | F | H | OC₂H₅ |
| 532. | 640. | 748. | 856. | C₅H₁₁ | H | H | H | H | OC₂H₅ |
| 533. | 641. | 749. | 857. | C₅H₁₁ | F | H | H | H | OC₂H₅ |
| 534. | 642. | 750. | 858. | C₅H₁₁ | F | H | F | H | OC₂H₅ |
| 535. | 643. | 751. | 859. | C₆H₁₃ | H | H | H | H | OC₂H₅ |
| 536. | 644. | 752. | 860. | C₆H₁₃ | F | H | H | H | OC₂H₅ |
| 537. | 645. | 753. | 861. | C₆H₁₃ | F | H | F | H | OC₂H₅ |
| 538. | 646. | 754. | 862. | C₇H₁₅ | H | H | H | H | OC₂H₅ |
| 539. | 647. | 755. | 863. | C₇H₁₅ | F | H | H | H | OC₂H₅ |
| 540. | 648. | 756. | 864. | C₇H₁₅ | F | H | F | H | OC₂H₅ |

### Beispiel 865

Eine Flüssigkristallmischung enthaltend

| | |
|---|---|
| BCH-3F.F | 10,80 % |
| BCH-5F.F | 9,00 % |
| ECCP-3OCF₃ | 4,50 % |
| ECCP-5OCF₃ | 4,50 % |
| CBC-33F | 1 ,80 % |
| CBC-53F | 1,80 % |
| CBC-55F | 1,80 % |
| PCH-5F | 9,00 % |
| PCH-6F | 7,20 % |
| PCH-7F | 5,40 % |
| CCP-2OCF₃ | 7,20 % |
| CCP-3OCF₃ | 10,80 % |
| CCP-4OCF₃ | 6,30 % |
| CCP-5OCF₃ | 9,90 % |
| Verbindung des Beispiels 1 | 10,00 % |

weist folgende Eigenschaften auf:

| | |
|---|---|
| Klärpunkt: | +86,0°C |
| Δε: | +5,6 |
| Δn: | +0,0924 |

### Beispiel 866

Eine Flüssigkristallmischung enthaltend

| | |
|---|---|
| CCH-3O1 | 11,23 % |
| CCH-3CF₃ | 6,42 % |
| CCH-5O1 | 8,82 % |
| CCP-2F.F.F | 8,02 % |
| CCP-3F.F.F | 10,42 % |
| CCP-5F.F.F | 4,01 % |
| CCPC-33 | 2,41 % |
| CCZU-2-F | 4,01 % |
| CCZU-3-F | 13,62 % |
| CCZU-5-F | 4,01 % |
| CH-33 | 2,41 % |
| CH-35 | 2,41 % |
| CH-43 | 2,41 % |
| Verbindung des Beispiels 1 | 19,80 % |

weist folgende Eigenschaften auf:

| | |
|---|---|
| Klärpunkt: | +72,0°C |
| Δn: | +0,0605 |

### Beispiel 867

Eine Flüssigkristallmischung enthaltend

| | |
|---|---|
| BCH-3F.F | 10,76 % |
| BCH-5F.F | 8,98 % |
| ECCP-30CF₃ | 4,49 % |
| ECCP-50CF₃ | 4,49 % |
| CBC-33F | 1,80 % |
| CBC-53F | 1,80 % |
| CBC-55F | 1,80 % |
| PCH-6F | 7,18 % |
| PCH-7F | 5,39 % |
| CCP-20CF₃ | 7,18 % |
| CCP-30CF₃ | 10,76 % |
| CCP-40CF₃ | 6,28 % |
| CCP-50CF₃ | 9,87 % |
| PCH-5F | 8,98 % |
| Verbindung des Beispiels 433 | 10,24 % |

weist folgende Eigenschaften auf:

| | |
|---|---|
| Klärpunkt: | +73,2°C |
| Δε: | +5,3 |
| Δn: | +0,0885 |

## Patentansprüche

1. Cyclobutanderivate der Formel I 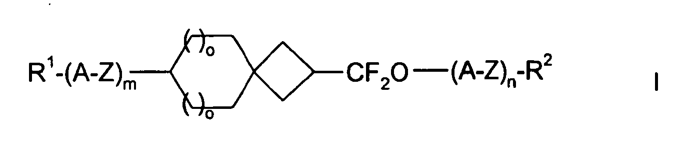 worin
R¹, R² jeweils unabhängig voneinander, gleich oder verschieden H, Halogen (F, Cl, Br, I) oder einen linearen oder verzweigten, gegebenenfalls chiralen, unsubstituierten, ein- oder mehrfach durch Halogen substituierten Alkyl- oder Alkoxyrest mit 1 bis 15 C-Atomen, worin auch eine oder mehrere CH₂-Gruppen jeweils unabhängig voneinander durch -O-, -S-, -CO-, -CO-O-, -O-CO-, -O-CO-O-, -CH=CH-, -CH=CF-, -CF=CF-, -C=C- oder -◇- so ersetzt sein können, dass Heteroatome nicht direkt miteinander verknüpft sind, -CN, -SCN, -SF₅, -SCF₃, -CF₃, -CF=CF₂, -CF₂CF₂CF₃, -OCF₃, -OCHF₂, -CF₂CH₂CF₃ oder -OCH₂CF₂CHFCF₃,
A jeweils unabhängig voneinander, gleich oder verschieden
a) trans-1,4-Cyclohexylen, worin auch eine oder mehrere nicht benachbarte CH₂-Gruppen durch -O- und/oder -S- ersetzt sein können und worin auch ein oder mehrere H-Atome durch F ersetzt sein können,
b) 1,4-Phenylen, worin eine oder zwei CH-Gruppen durch N ersetzt sein können und worin auch ein oder mehrere H-Atome durch Halogen (F, Cl, Br, I), -CN, -CH₃, -CHF₂, -CH₂F, -OCH₃, -OCHF₂ oder -OCF₃ ersetzt sein können,
c) einen Rest aus der Gruppe Bicyclo[1.1.1]pentan-1,3-diyl, Bicyclo[2.2.2]-octan-1,4-diyl, Spiro[3.3]heptan-2,6-diyl, Naphthalin-2,6-diyl, Decahydronaphthalin-2,6-diyl, 1,2,3,4-Tetrahydronaphthalin-2,6-diyl und Piperidin-1,4-diyl, oder
d) 1,4-Cyclohexenylen,
Z jeweils unabhängig voneinander, gleich oder verschieden -O-, -CH₂O-, -OCH₂-, -CO-O-, -O-CO-, -CF₂O-, -OCF₂-, -CF₂CF₂-, -CH₂CF₂-, -CF₂CH₂-, -CH₂CH₂-, -CH=CH-, -CH=CF-, -CF=CH-, -CF=CF-, -CF=CF-COO-, -O-CO-CF=CF-, -C≡C- oder eine Einfachbindung,
m, n unabhängig voneinander, gleich oder verschieden 0, 1 oder 2, und
o 0 oder 1,
bedeuten.

2. Verbindungen gemäß Anspruch 1, **dadurch gekennzeichnet, dass** beide o 0 bedeuten.

3. Verbindungen gemäß Anspruch 1, **dadurch gekennzeichnet, dass** beide o 1 bedeuten.

4. Verbindungen gemäß Anspruch 2, **dadurch gekennzeichnet, dass** sie eine der folgenden Formeln aufweisen: 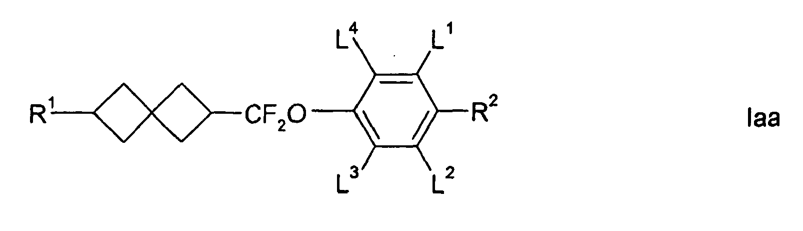 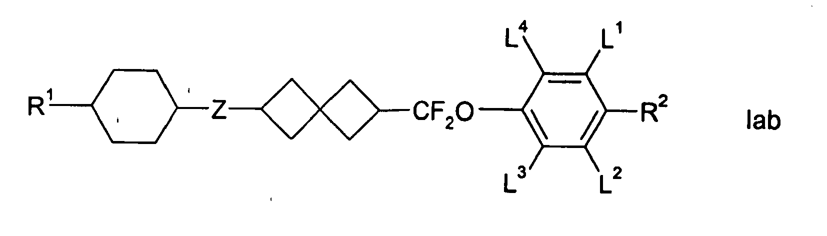 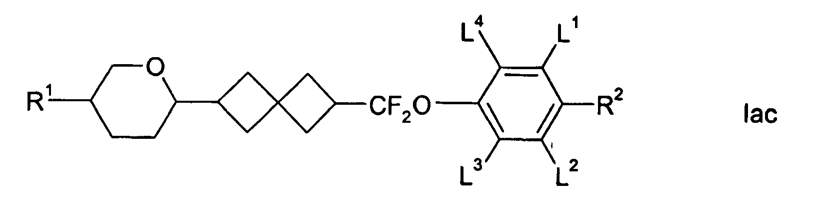 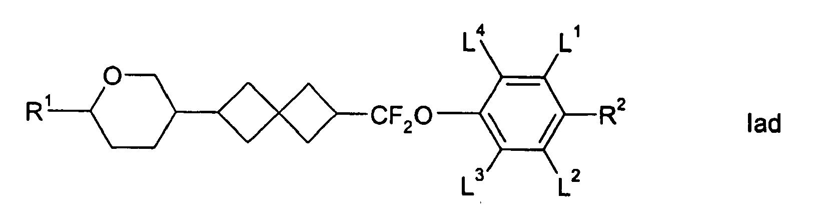 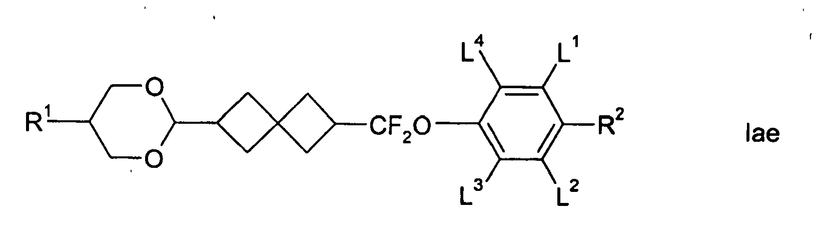 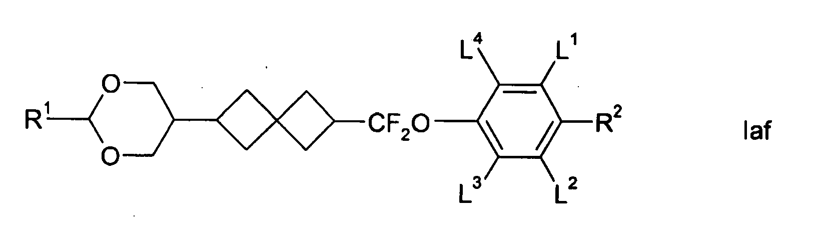 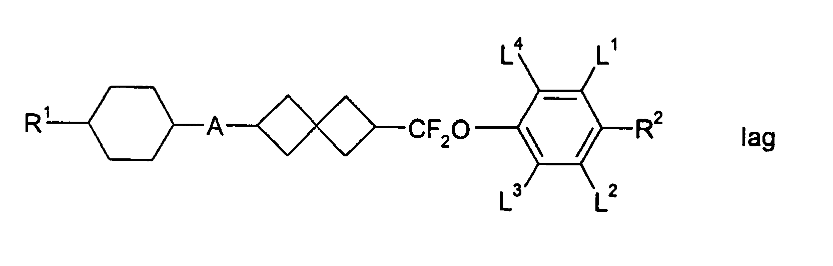 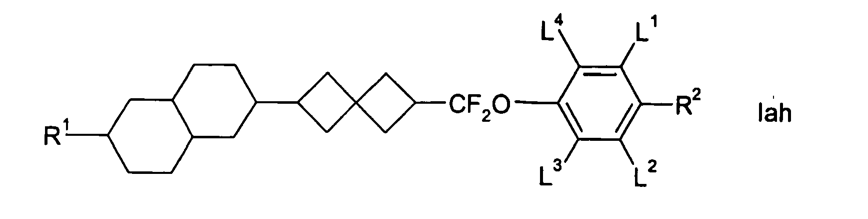 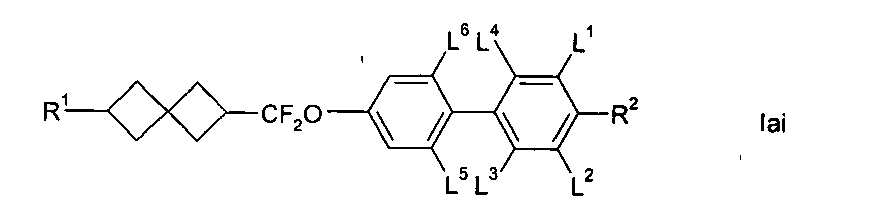 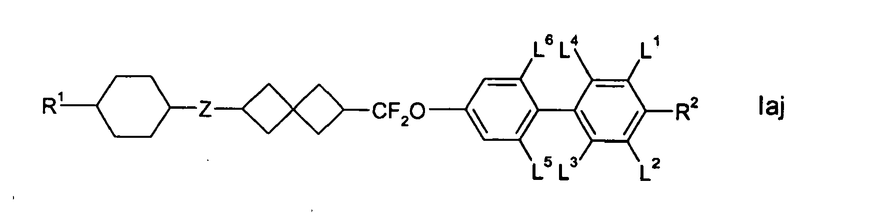 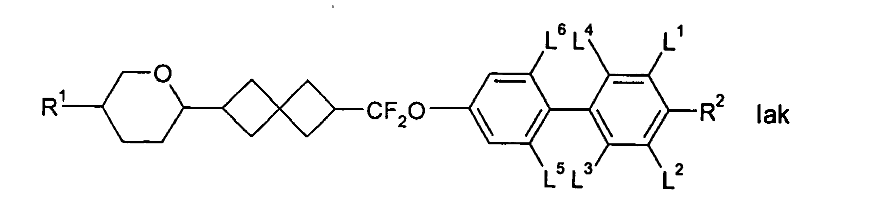 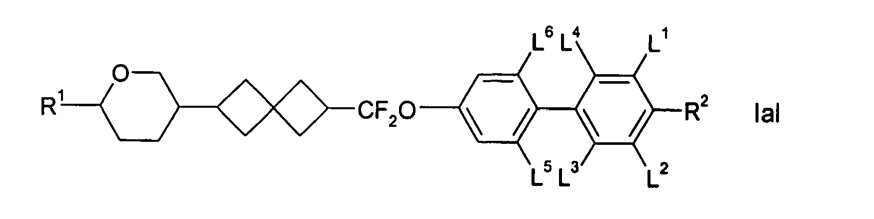 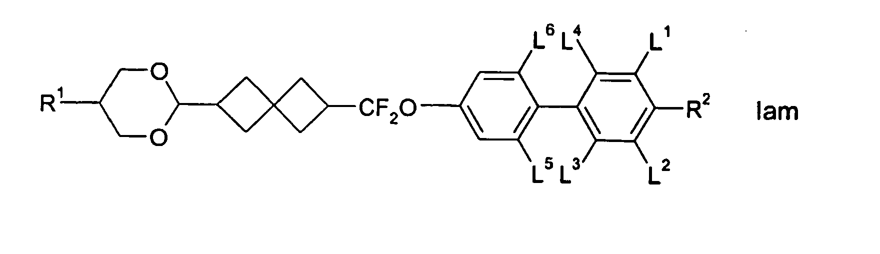 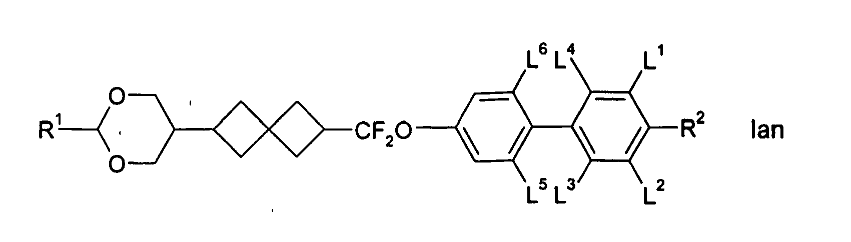 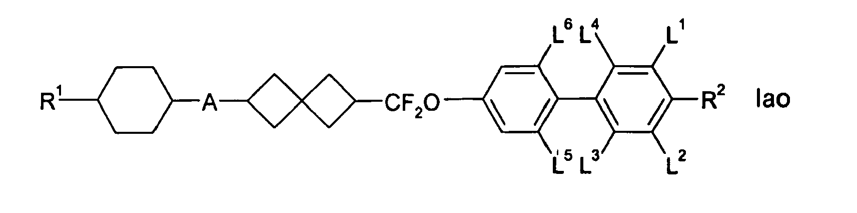 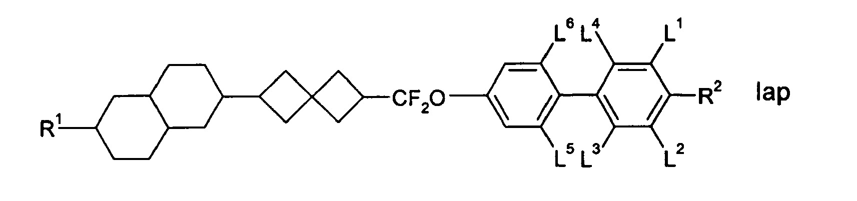 worin L¹, L², L³, L⁴, L⁵ und L⁶ unabhängig voneinander, gleich oder verschieden H oder F bedeuten.

5. Verbindungen gemäß Anspruch 3, **dadurch gekennzeichnet, dass** sie eine der folgenden Formeln aufweisen: 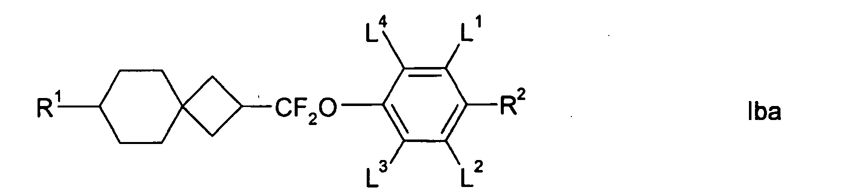 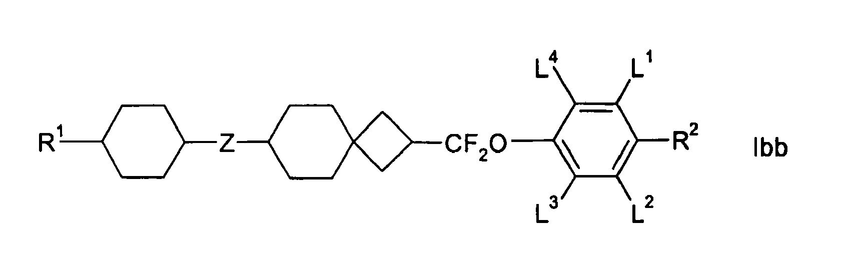 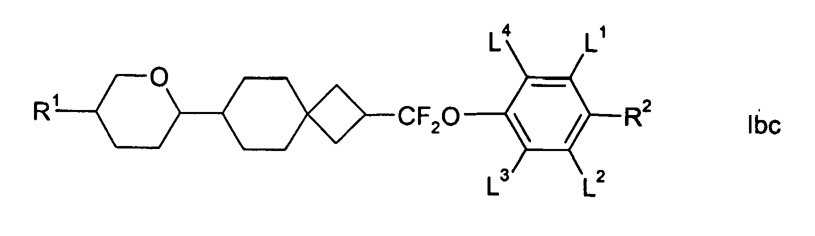 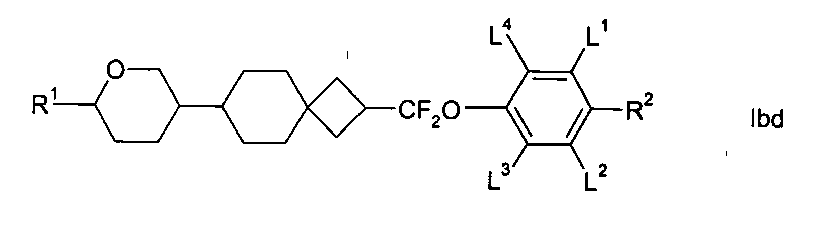 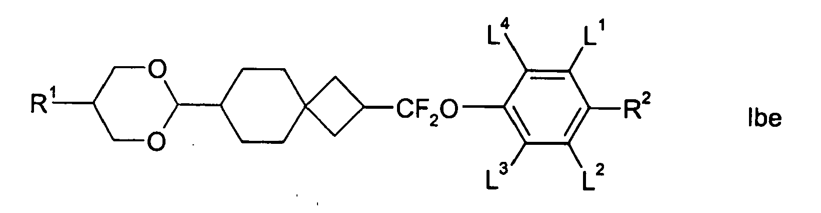 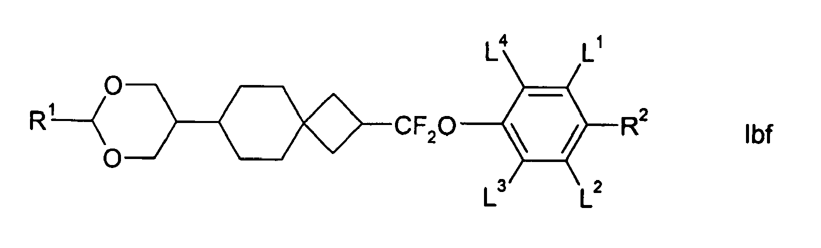 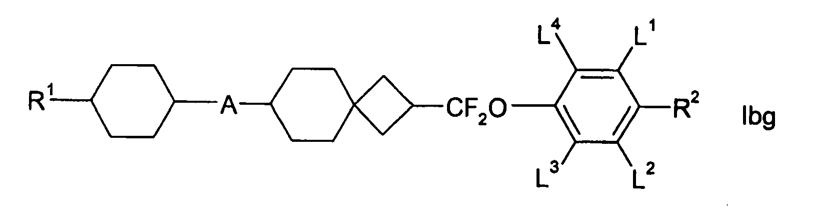 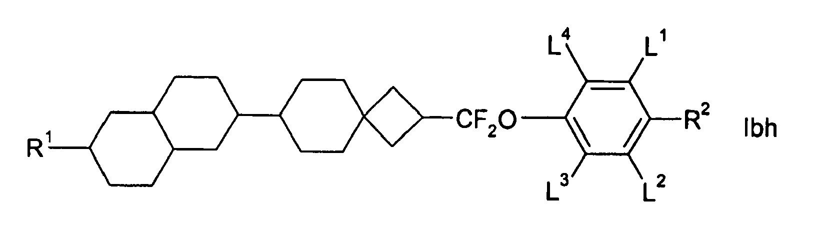 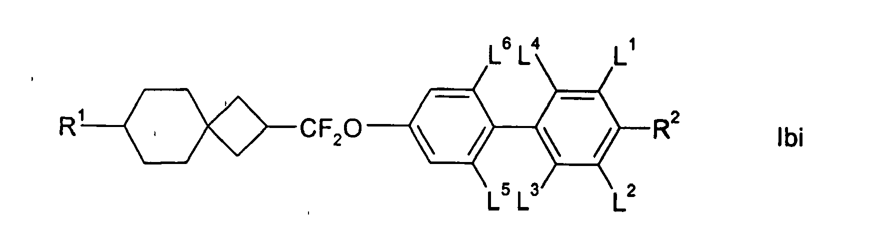 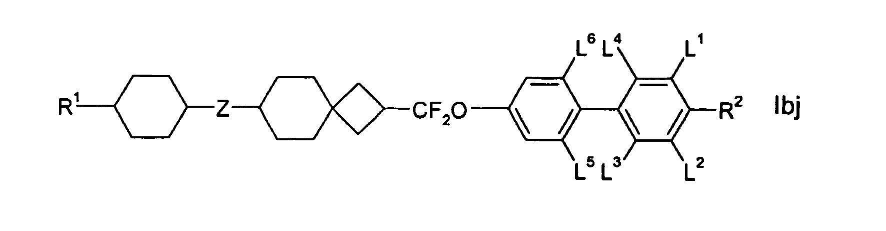 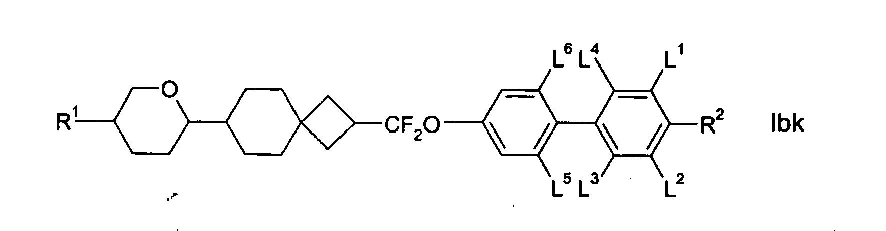 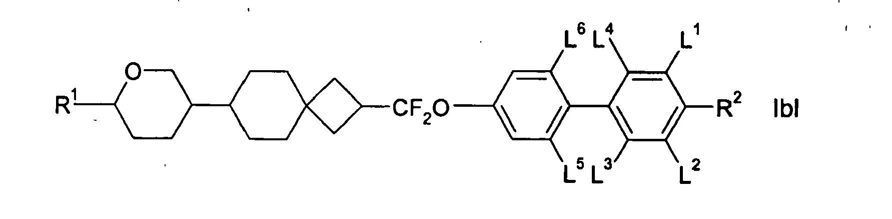 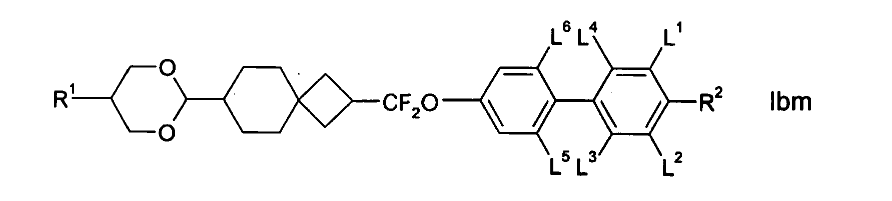 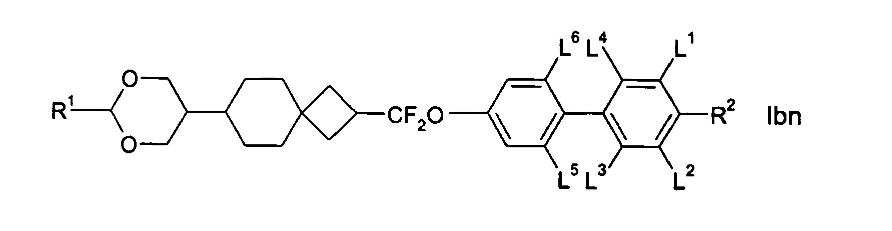 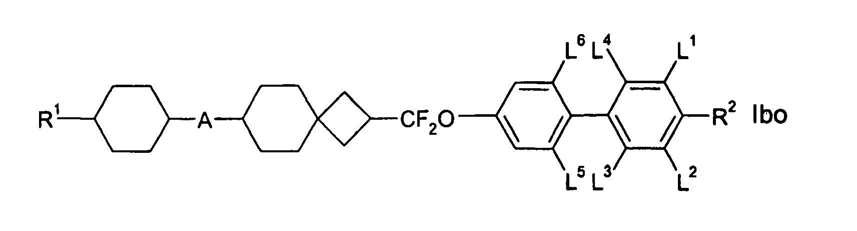 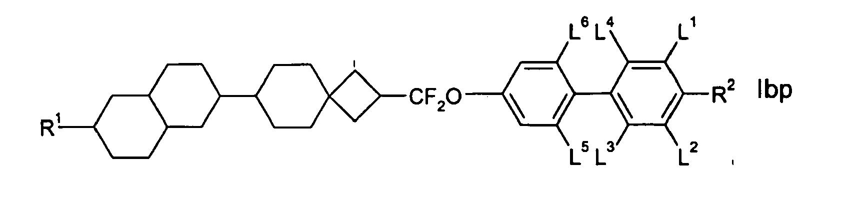 worin L¹, L², L³, L⁴, L⁵ und L⁶ unabhängig voneinander, gleich oder verschieden H oder F bedeuten.

6. Verbindungen gemäß mindestens einem der vorhergehenden Ansprüche, **dadurch gekennzeichnet, dass** R¹ H oder einen linearen Alkylrest mit 1 bis 10 C-Atomen bedeutet.

7. Verbindungen gemäß mindestens einem der vorhergehenden Ansprüche, **dadurch gekennzeichnet, dass** R² H, einen linearen Alkoxyrest mit 1 bis 10 C-Atomen, -F, -Cl, -CF₃, -OCF₃, -OCHF₂, -CN oder -SF₅ bedeutet.

8. Verwendung von Verbindungen der Formel I gemäß mindestens einem der vorhergehenden Ansprüche als Komponente(n) in flüssigkristallinen Medien.

9. Flüssigkristallines Medium mit mindestens zwei flüssigkristallinen Komponenten, **dadurch gekennzeichnet, dass** es mindestens eine Verbindung der Formel I gemäß mindestens einem der Ansprüche 1 bis 7 enthält.

10. Flüssigkristall-Anzeigeelement, **dadurch gekennzeichnet, dass** es als Dielektrikum ein flüssigkristallines Medium gemäß Anspruch 9 enthält.

11. Reflektives oder transflektives Flüssigkristall-Anzeigeelement, **dadurch gekennzeichnet, dass** es als Dielektrikum ein flüssigkristallines Medium gemäß Anspruch 9 enthält.

12. Elektrooptisches Anzeigeelement, **dadurch gekennzeichnet, dass** es als Dielektrikum ein flüssigkristallines Medium gemäß Anspruch 9 enthält.

## Claims

1. Cyclobutane derivatives of the formula I 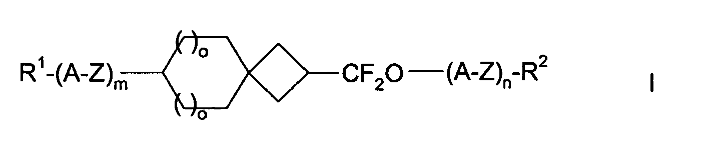 in which
R¹, R² each, independently of one another, identically or differently, denote H, halogen (F, Cl, Br, I) or a linear or branched, optionally chiral alkyl or alkoxy radical having 1 to 15 C atoms which is unsubstituted or mono- or polysubstituted by halogen and in which one or more CH₂ groups may each be replaced, independently of one another, by -O-, -S-, -CO-, -CO-O-, -O-CO-, -O-CO-O-, -CH=CH-, -CH=CF-, -CF=CF-, -C≡C- or -◇- in such a way that heteroatoms are not linked directly to one another, -CN, -SCN, -SF₅, -SCF₃, -CF₃, -CF=CF₂, -CF₂CF₂CF₃, -OCF₃, -OCHF₂, -CF₂CH₂CF₃ or -OCH₂CF₂CHFCF₃,
A in each case, independently of one another, identically or differently, denotes
a) trans-1,4-cyclohexylene, in which, in addition, one or more non-adjacent CH₂ groups may be replaced by -O- and/or -S- and in which, in addition, one or more H atoms may be replaced by F,
b) 1,4-phenylene, in which one or two CH groups may be replaced by N and in which, in addition, one or more H atoms may be replaced by halogen (F, Cl, Br, I), -CN, -CH₃, -CHF₂, -CH₂F, -OCH₃, -OCHF₂ or -OCF₃,
c) a radical from the group bicyclo[1.1.1]pentane-1,3-diyl, bicyclo[2.2.2]octane-1,4-diyl, spiro[3.3]heptane-2,6-diyl, naphthalene-2,6-diyl, decahydronaphthalene-2,6-diyl, 1,2,3,4-tetrahydronaphthalene-2,6-diyl and piperidine-1,4-diyl, or
d) 1,4-cyclohexenylene,
Z in each case, independently of one another, identically or differently, denotes -O-, -CH₂O-, -OCH₂-, -CO-O-, -O-CO-, -CF₂O-, -OCF₂-, -CF₂CF₂-, -CH₂CF₂-, -CF₂CH₂-, -CH₂CH₂-, -CH=CH-, -CH=CF-, -CF=CH-, -CF=CF-, -CF=CF-COO-, -O-CO-CF=CF-, -C≡C- or a single bond,
m, n, independently of one another, identically or differently, denote 0, 1 or 2, and
o denotes 0 or 1.

2. Compounds according to Claim 1, **characterised in that** both o denote 0.

3. Compounds according to Claim 1, **characterised in that** both o denote 1.

4. Compounds according to Claim 2, **characterised in that** they have one of the following formulae: 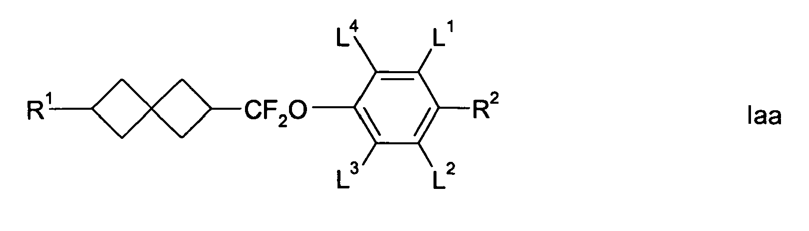 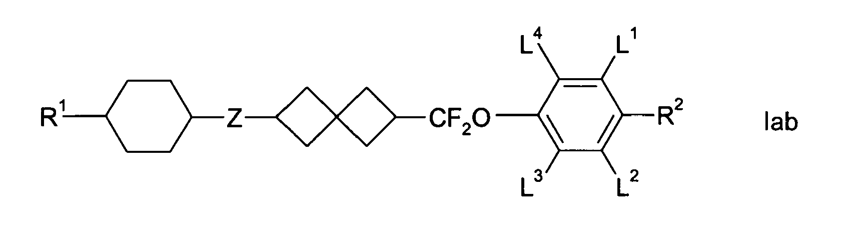 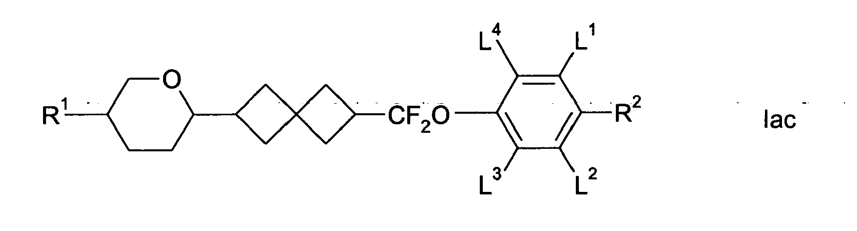 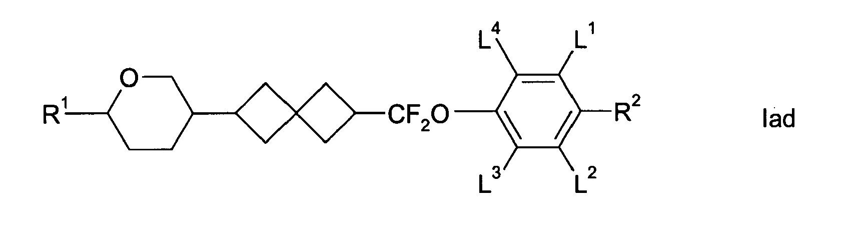 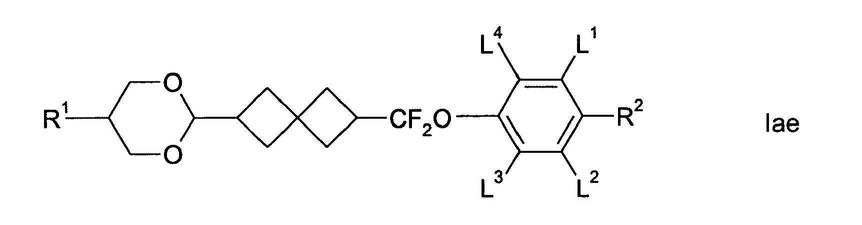 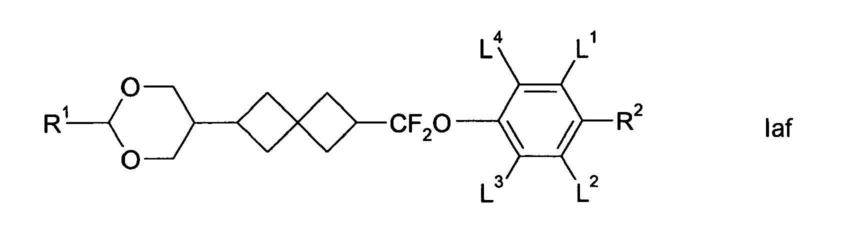 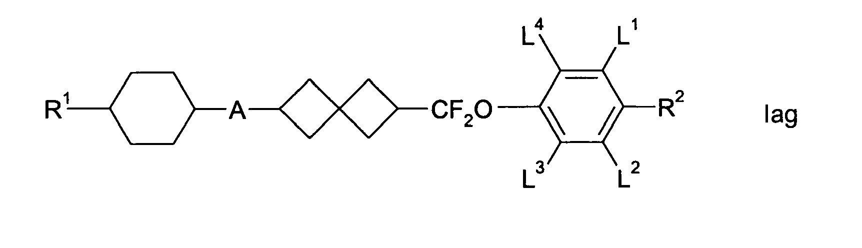 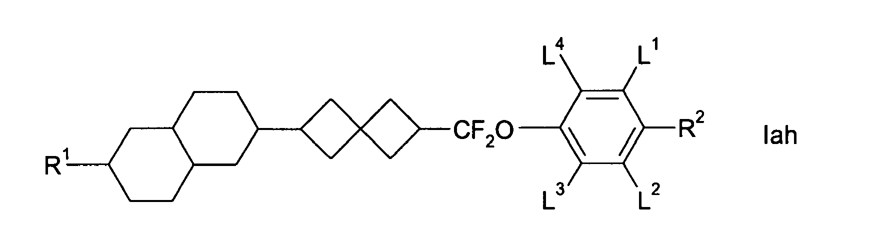 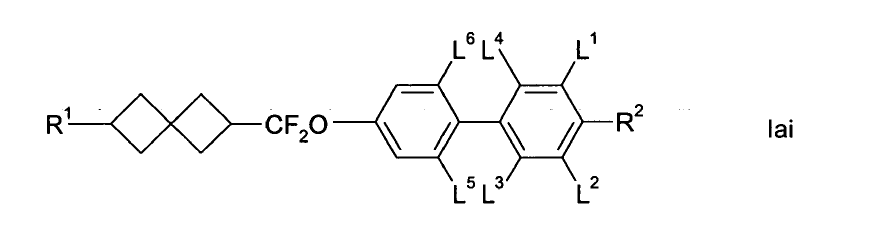 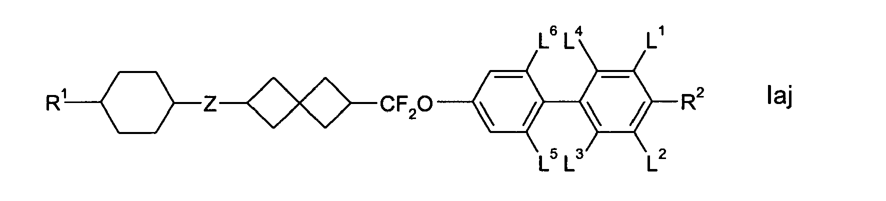 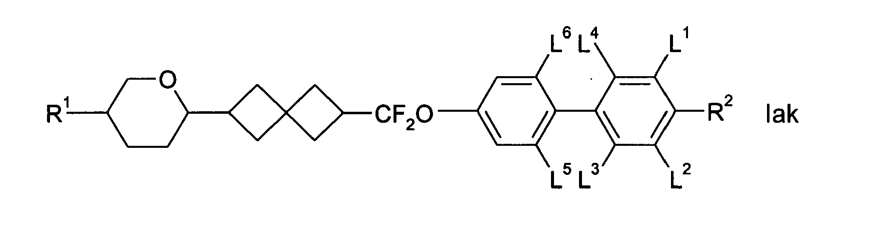 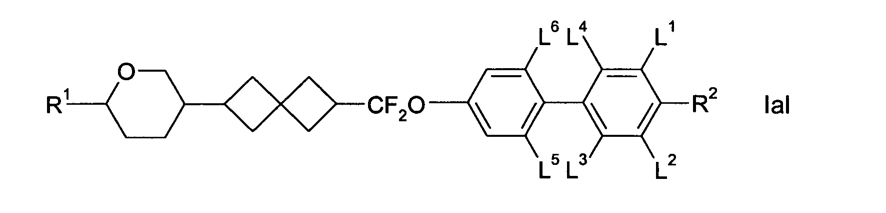 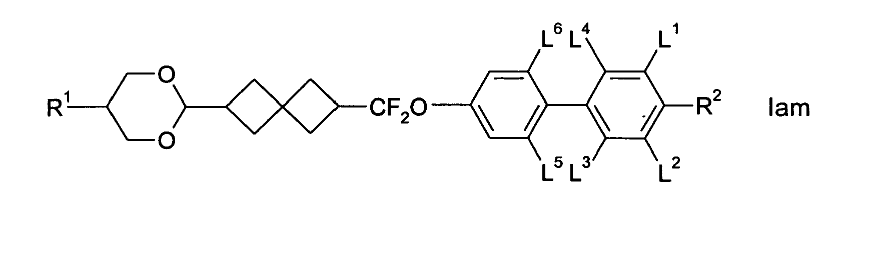 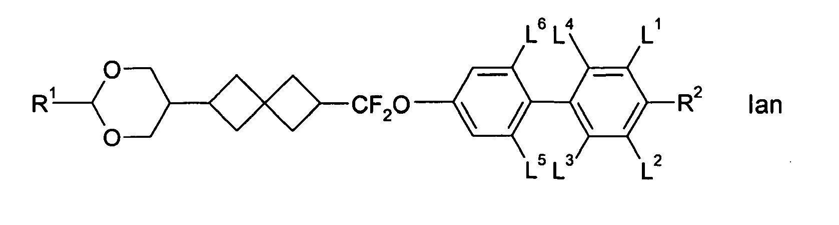 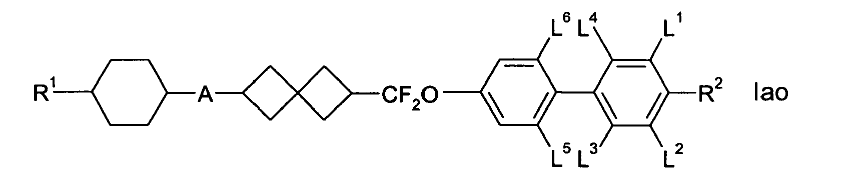 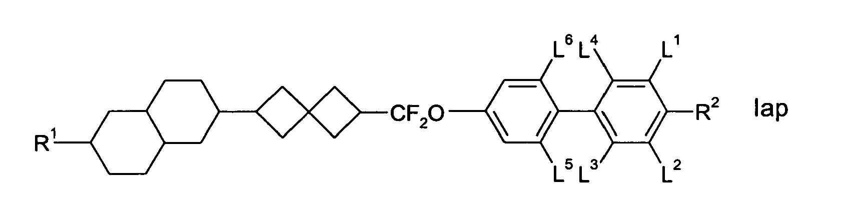 in which L¹, L², L³, L⁴, L⁵ and L⁶, independently of one another, identically or differently, denote H or F.

5. Compounds according to Claim 3, **characterised in that** they have one of the following formulae: 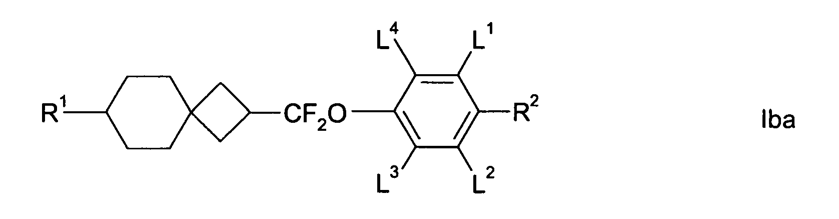 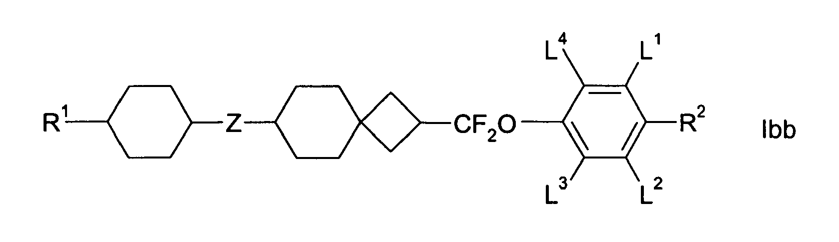 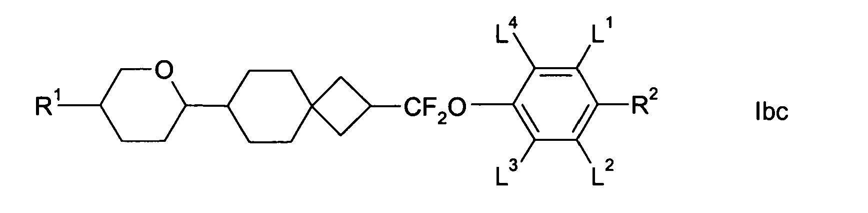 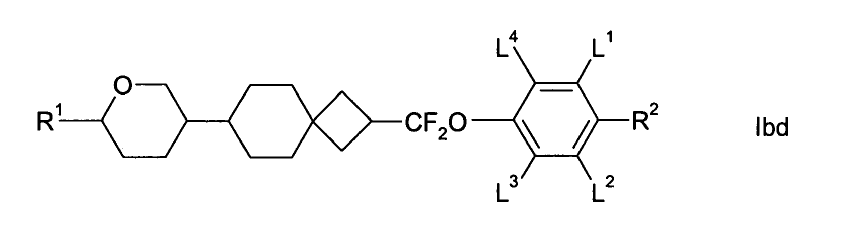 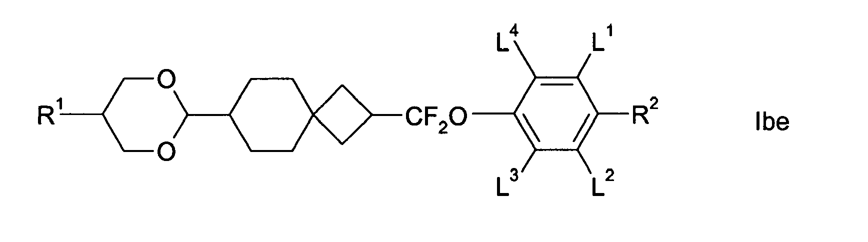 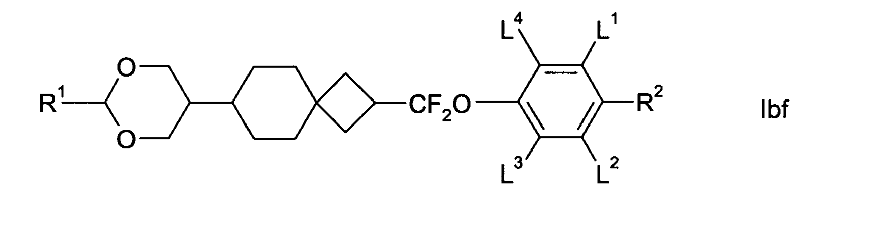 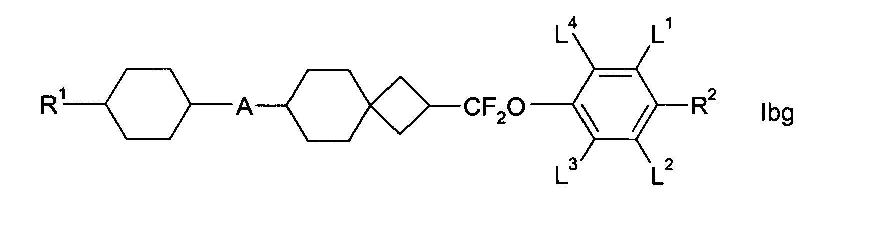 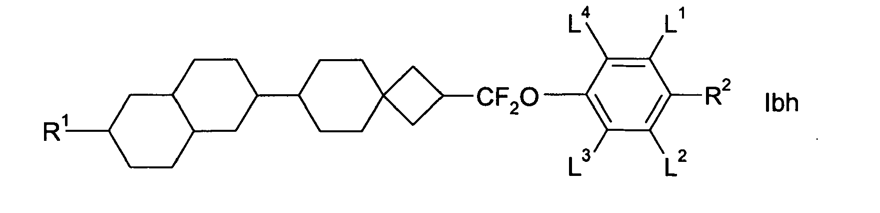 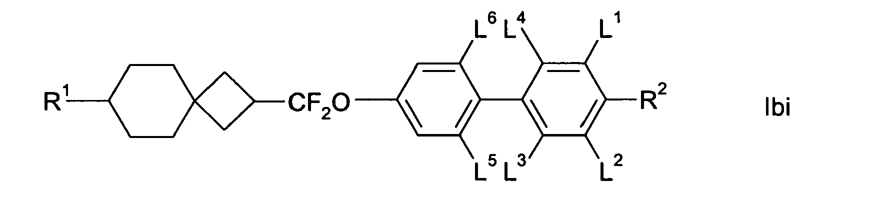 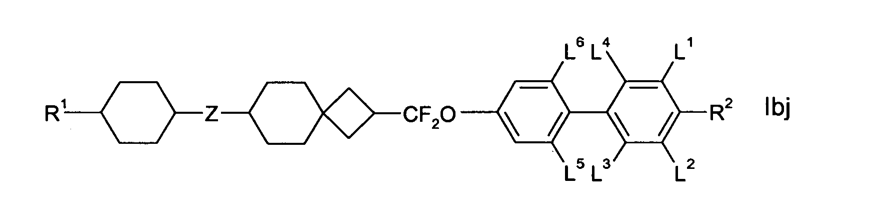 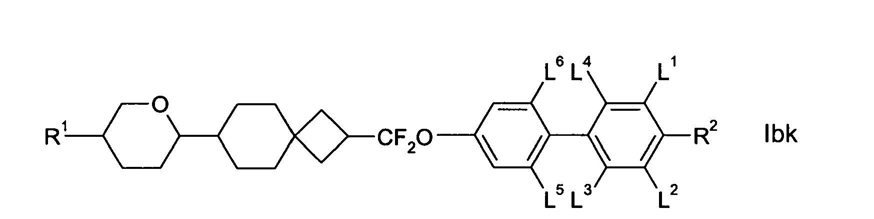 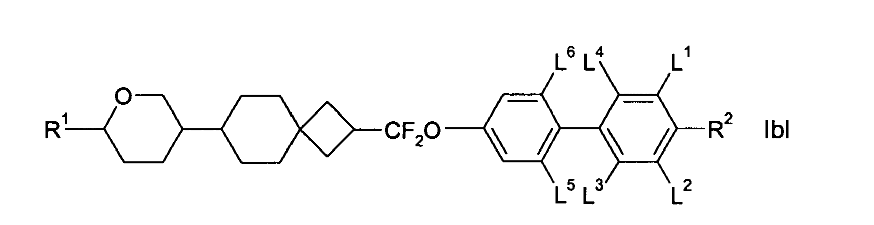 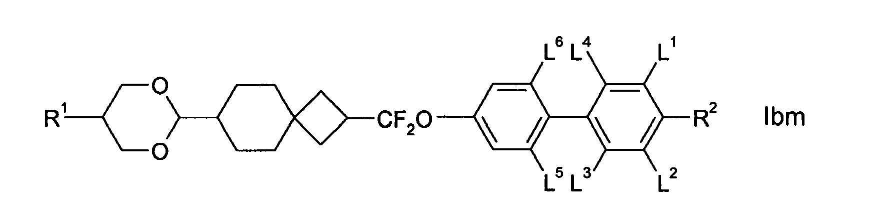 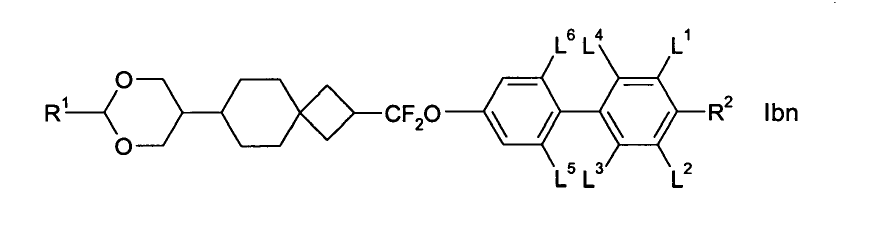 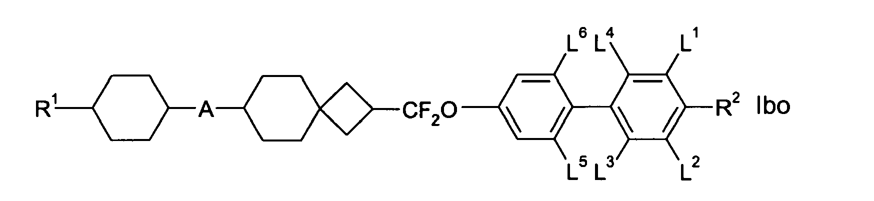 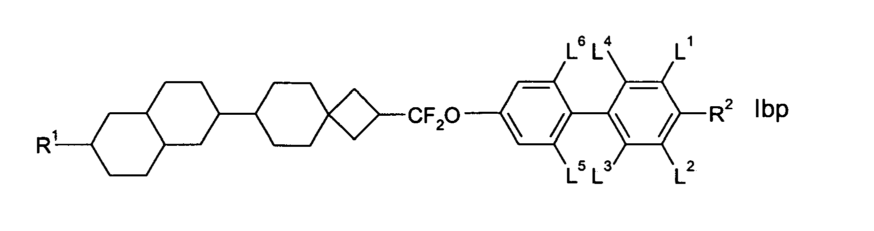 in which L¹, L², L³, L⁴, L⁵ and L⁶, independently of one another, identically or differently, denote H or F.

6. Compounds according to at least one of the preceding claims, **characterised in that** R¹ denotes H or a linear alkyl radical having 1 to 10 C atoms.

7. Compounds according to at least one of the preceding claims, **characterised in that** R² denotes H, a linear alkoxy radical having 1 to 10 C atoms, -F, -Cl, -CF₃, -OCF₃, -OCHF₂, -CN or -SF₅.

8. Use of compounds of the formula I according to at least one of the preceding claims as component(s) of liquid-crystalline media.

9. Liquid-crystalline medium having at least two liquid-crystalline components, **characterised in that** it comprises at least one compound of the formula I according to at least one of Claims 1 to 7.

10. Liquid-crystal display element, **characterised in that** it contains, as dielectric, a liquid-crystalline medium according to Claim 9.

11. Reflective or transflective liquid-crystal display element, **characterised in that** it contains, as dielectric, a liquid-crystalline medium according to Claim 9.

12. Electro-optical display element, **characterised in that** it contains, as dielectric, a liquid-crystalline medium according to Claim 9.

## Revendications

1. Dérivés de cyclobutane de la formule I 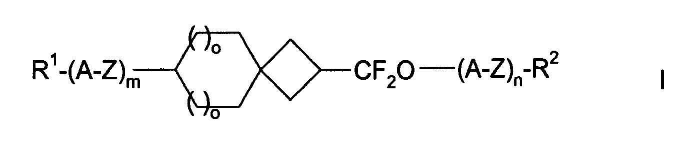 dans laquelle
R¹, R² chacun indépendamment l'un de l'autre, de manière identique ou différente, représentent H, halogène (F, Cl, Br, I) ou un radical alkyle ou alcoxy linéaire ou ramifié, en option chiral comportant de 1 à 15 atomes de C qui est non substitué ou mono- ou polysubstitué par halogène et où un ou plusieurs groupes CH₂ peuvent chacun être remplacés, indépendamment les uns des autres, par -O-, -S-, -CO-, -CO-O-, -O-CO-, -O-CO-O-, -CH=CH-, -CH=CF-, -CF=CF-, -C≡C- ou -◇- de telle sorte que des hétéroatomes ne soient pas liés directement les uns aux autres, -CN, -SCN, -SF₅, -SCF₃, -CF₃, -CF=CF₂, -CF₂CF₂CF₃, -OCF₃, -OCHF₂, -CF₂CH₂CF₃ ou -OCH₂CF₂CHFCF₃,
A dans chaque cas indépendamment les uns des autres, de manière identique ou différente, représente
a) trans-1,4-cyclohexylène, où, en plus, un ou plusieurs groupes CH₂ non adjacents peuvent être remplacés par -O- et/ou -S- et où, en plus, un ou plusieurs atomes de H peuvent être remplacés par F,
b) 1,4-phénylène, où un ou deux groupes CH peuvent être remplacés par N et où, en plus, un ou plusieurs atomes de H peuvent être remplacés par halogène (F, Cl, Br, I), -CN, -CH₃, -CHF₂, -CH₂F, -OCH₃, -OCHF₂ ou -OCF₃,
c) un radical pris parmi le groupe bicyclo[1.1.1]pentane-1,3-diyle, bicyclo[2.2.2]octane-1,4-diyle, spiro[3.3]heptane-2,6-diyle, naphthalene-2,6-diyle, décahydronaphthalène-2,6-diyle, 1,2,3,4-tétrahydronaphthalène-2,6-diyle et pipéridine-1,4-diyle, ou
d) 1,4-cyclohexénylène,
Z dans chaque cas indépendamment les uns des autres, de manière identique ou différente, représente -O-, -CH₂O-, -OCH₂-, -CO-O-, -O-CO-, -CF₂O-, -OCF₂-, -CF₂CF₂-, -CH₂CF₂-, -CF₂CH₂-, -CH₂CH₂-, -CH=CH-, -CH=CF-, -CF=CH-, -CF=CF-, -CF=CF-COO-, -O-CO-CF=CF-, -C=C- ou une liaison simple,
m, n, indépendamment l'un de l'autre, de manière identique ou différente, représentent 0, 1 ou 2, et
o représente 0 ou 1.

2. Composés selon la revendication 1, **caractérisés en ce que** les deux o représentent 0.

3. Composés selon la revendication 1, **caractérisés en ce que** les deux o représentent 1.

4. Composés selon la revendication 2, **caractérisés en ce qu'**ils présentent l'une des formules qui suivent : 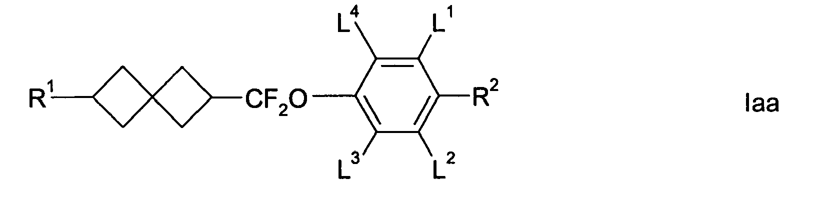 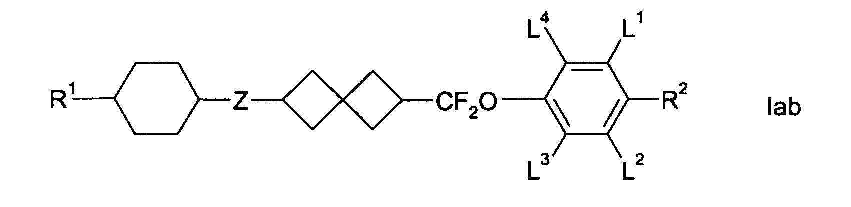 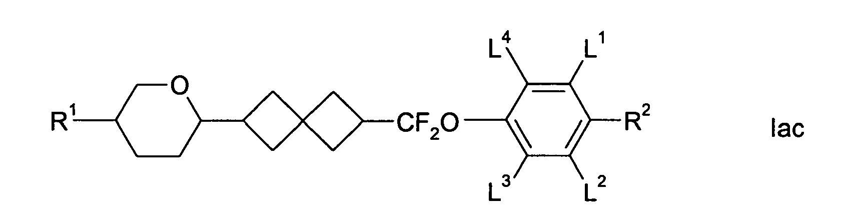 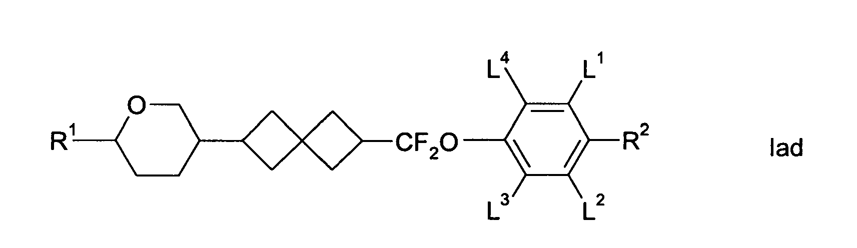 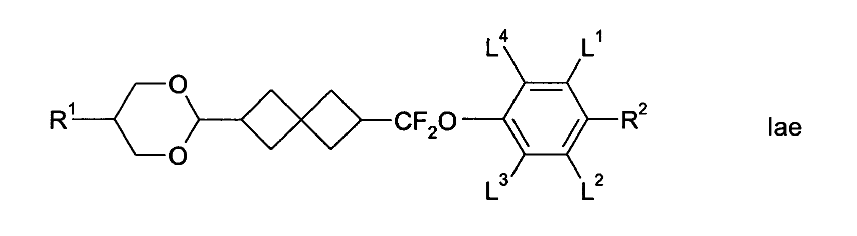 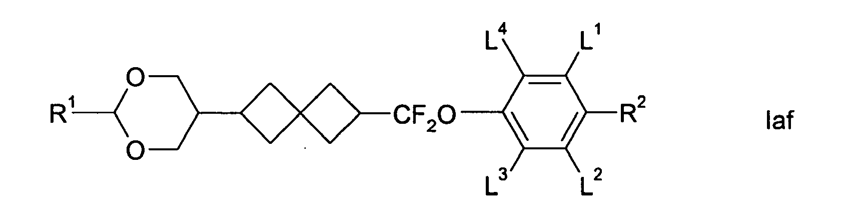 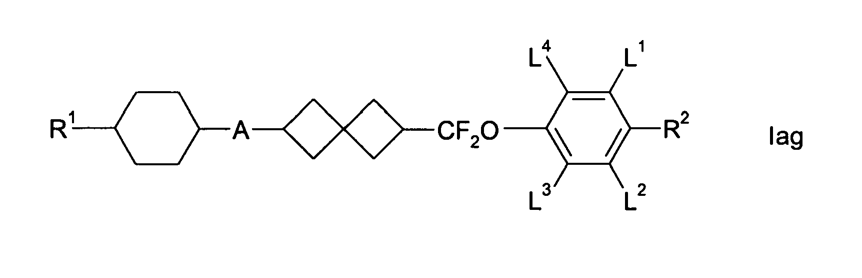 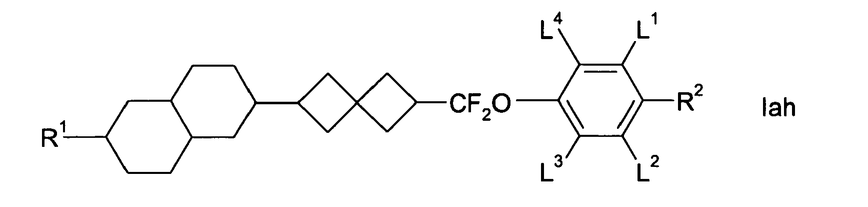 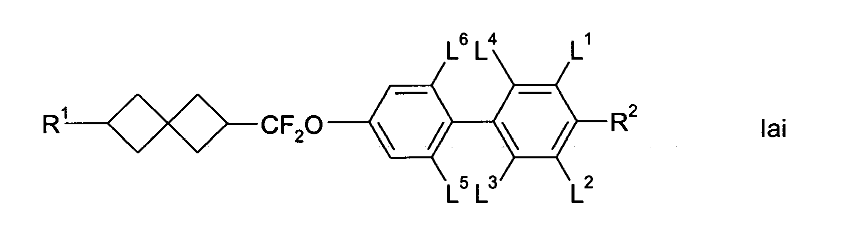 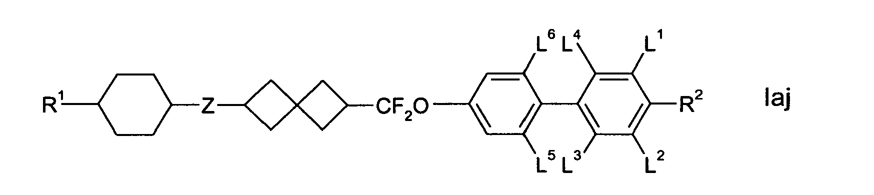 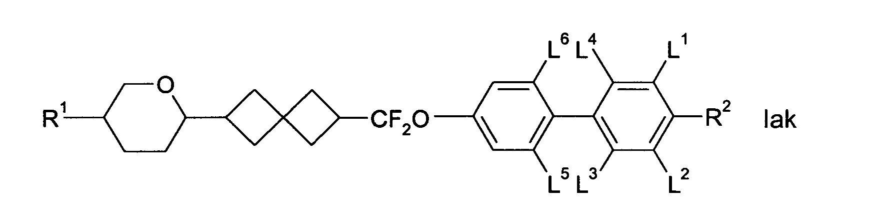 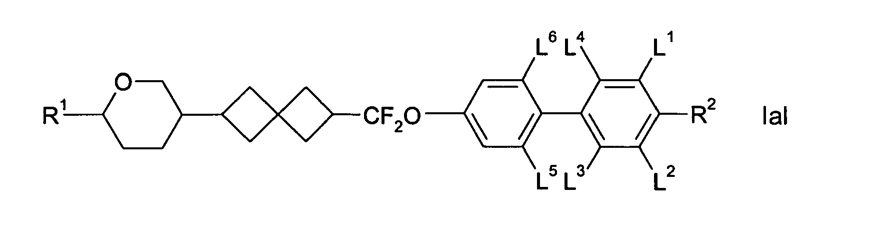 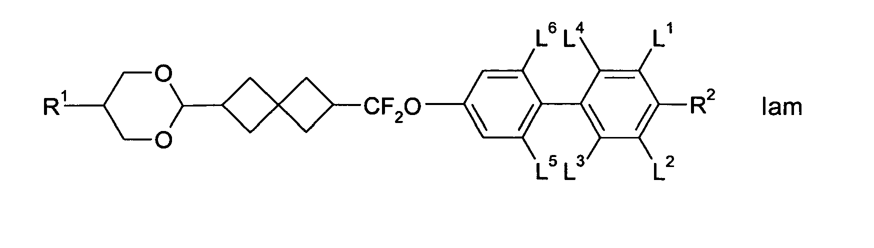 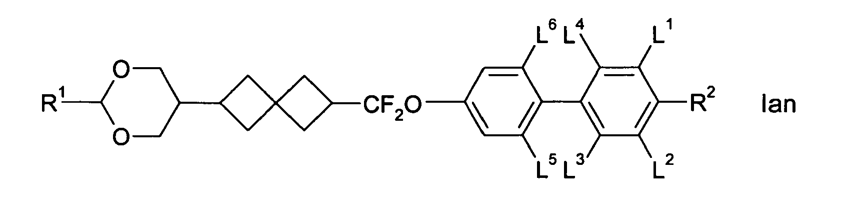 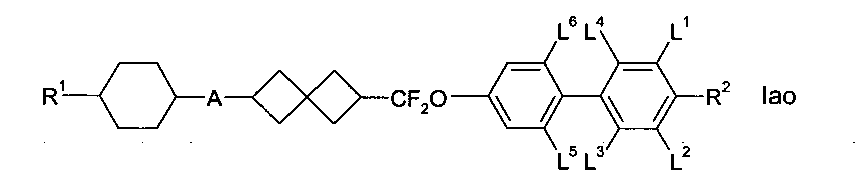 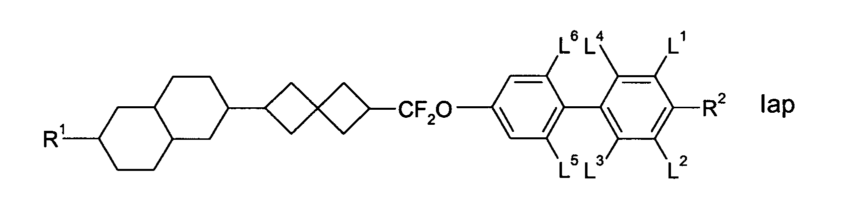 dans lesquelles L¹, L², L³, L⁴, L⁵ et L⁶, indépendamment les uns des autres, de manière identique ou différente, représentent H ou F.

5. Composés selon la revendication 3, **caractérisés en ce qu'**ils présentent l'une des formules qui suivent : 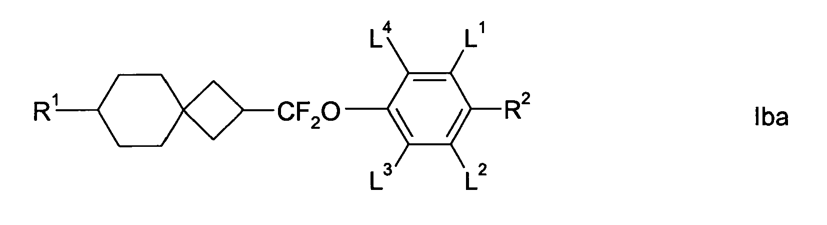 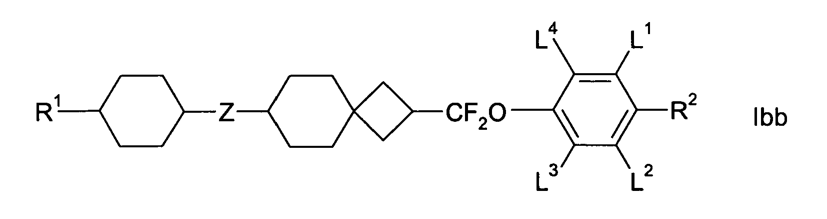 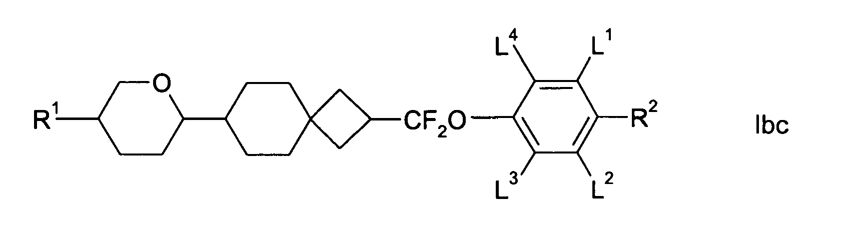 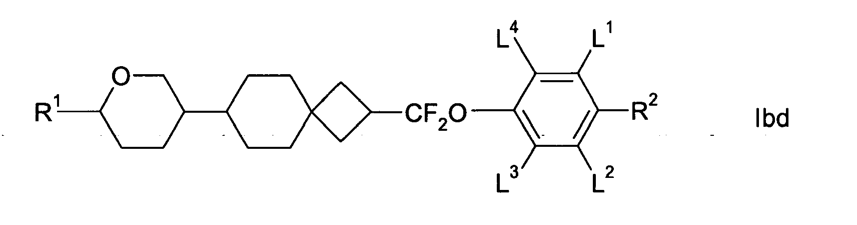 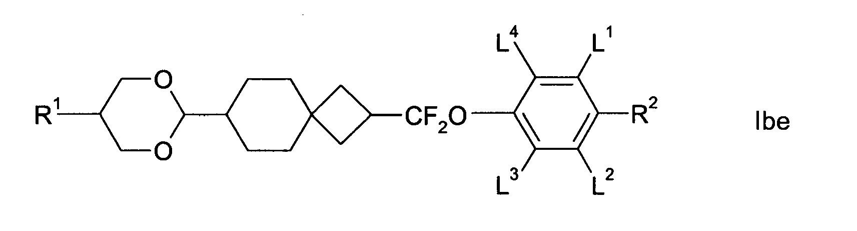 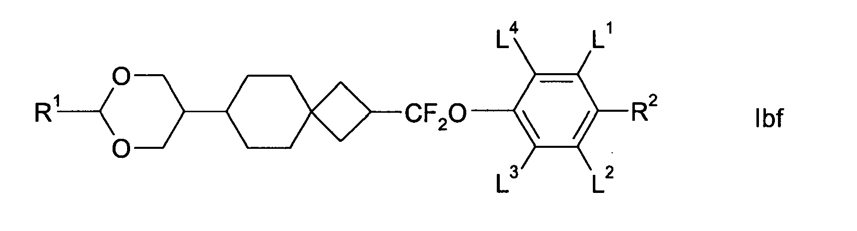 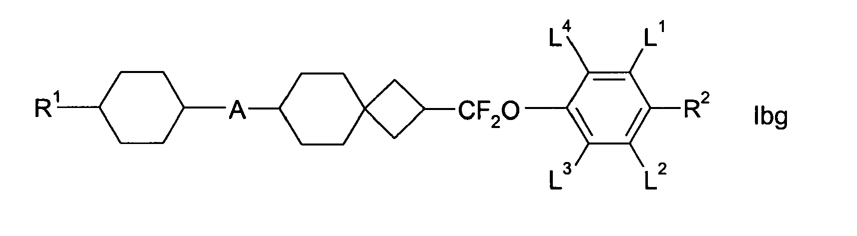 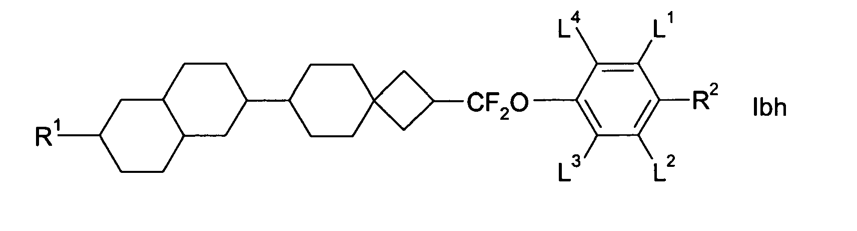 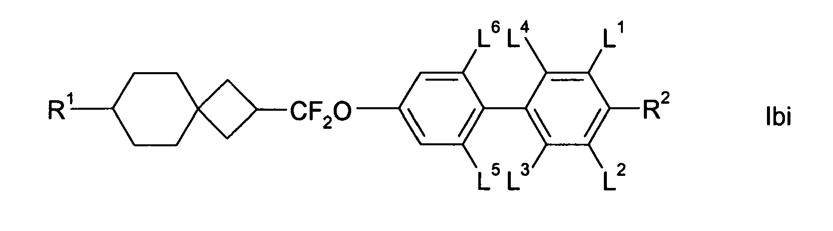 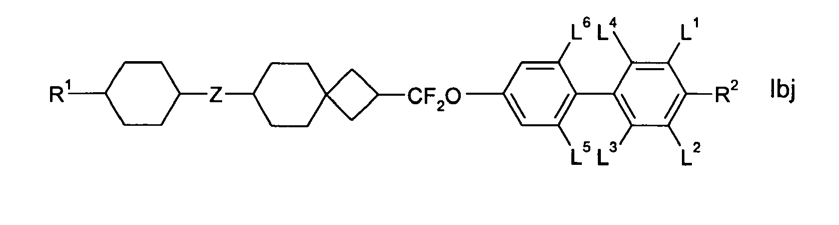 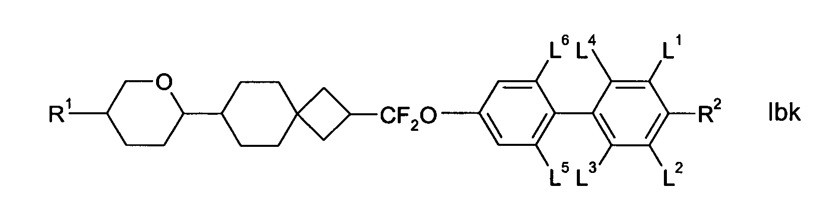 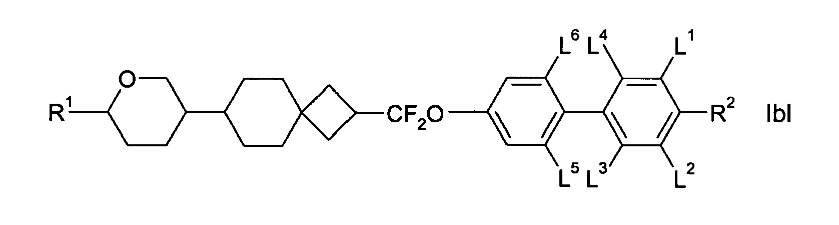 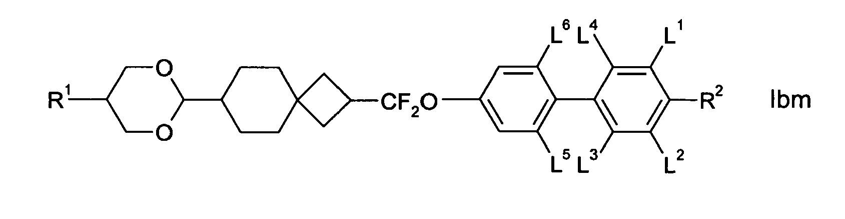 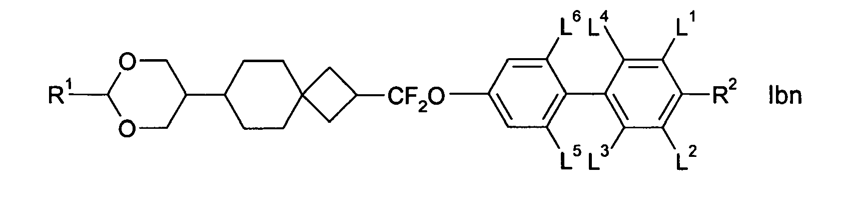 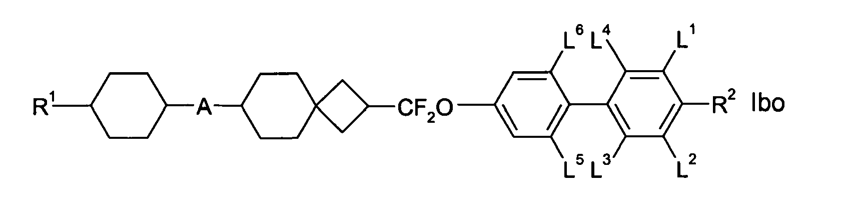 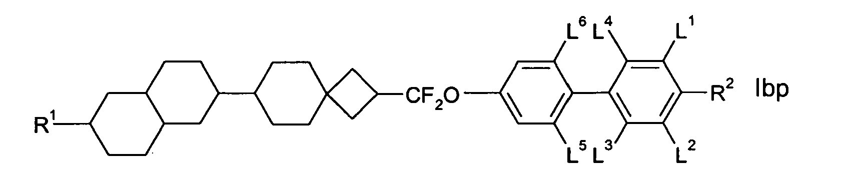 dans lesquelles L¹, L², L³, L⁴, L⁵ et L⁶, indépendamment les uns des autres, de manière identique ou différente, représentent H ou F.

6. Composés selon au moins l'une des revendications précédentes, **caractérisés en ce que** R¹ représente H ou un radical alkyle linéaire comportant de 1 à 10 atomes de C.

7. Composés selon au moins l'une des revendications précédentes, **caractérisés en ce que** R² représente H, un radical alcoxy linéaire comportant de 1 à 10 atomes de C, -F, -Cl, -CF₃, -OCF₃, -OCHF₂, -CN ou -SF₅.

8. Utilisation de composés de la formule I selon au moins l'une des revendications précédentes en tant que composant(s) d'un milieu de cristal liquide.

9. Milieu de cristal liquide comportant au moins deux composants de cristal liquide, **caractérisé en ce qu'**il comprend au moins un composé de la formule I selon au moins l'une des revendications 1 à 7.

10. Elément d'affichage à cristal liquide, **caractérisé en ce qu'**il contient, en tant que diélectrique, un milieu de cristal liquide selon la revendication 9.

11. Elément d'affichage à cristal liquide réfléchissant ou transflectif, **caractérisé en ce qu'**il contient, en tant que diélectrique, un milieu de cristal liquide selon la revendication 9.

12. Elément d'affichage électro-optique, **caractérisé en ce qu'**il contient, en tant que diélectrique, un milieu de cristal liquide selon la revendication 9.
